# EUROPEAN PATENT SPECIFICATION

(11) **EP 1 451 207 B1**
(45) Date of publication and mention of the grant of the patent: **26.08.2009**
(21) Application number: 02803304.1
(22) Date of filing: 05.11.2002
(51) Int. Cl.: G01N 33/68, G06F 19/00, H01J 49/04, H01J 49/16, H01J 49/40

(54) **LABELING REAGENT AND METHODS OF USE**
MARKIERUNGSREAGENS UND ANWENDUNGSVERFAHREN
REACTIF DE MARQUAGE ET SES PROCEDES D'UTILISATION

(30) Priority: 05.11.2001 US 332988 P; 03.06.2002 US 385835 P; 12.09.2002 US 410382 P
(43) Date of publication of application: 01.09.2004
(73) Proprietor: IRM LLC, Hamilton HM 11 (BM)
(72) Inventor: PETERS, Eric, C., Carlsbad, CA 92009 (US); BROCK, Ansgar, San Diego, CA 92131 (US); ERICSON, Christer, San Diego, CA 92122 (US)
(74) Representative: Thomson, Paul Anthony
(86) International application number: PCT/US2002/035581
(87) International publication number: WO 2003/056299

(56) References cited:
- EP-A- 0 097 031
- US-B1- 6 271 037
- US-B1- 6 489 608
- PETERS E C ET AL: "A NOVEL MULTIFUNCTIONAL LABELING REAGENT FOR ENHANCED PROTEIN CHARACTERIZATION WITH MASS SPECTROMETRY" RAPID COMMUNICATIONS IN MASS SPECTROMETRY, HEYDEN, LONDON, GB, vol. 15, no. 24, 2001, pages 2387-2392, XP001105041 ISSN: 0951-4198
- LEVINE L M ET AL: "Measurement of specific protease activity utilizing fluorescence polarization" ANALYTICAL BIOCHEMISTRY, ACADEMIC PRESS, SAN DIEGO, CA, US, vol. 247, no. 1, 1997, pages 83-88, XP002228468 ISSN: 0003-2697
- ZERKOWSKI E.A.: "Triazine conjugates for multiple site-specific labeling of peptides" PROTEIN AND PEPTIDE LETTERS, vol. 8, no. 2, April 2001 (2001-04), pages 123-129, XP002333774
- RIVIER J ET AL: "GONADOTROPIN RELEASING HORMONE ANTAGONISTS:NOVEL STRUCTURES INCORPORATING NW-CYANO MODIFIED GUANIDINE MOIETIES" BIOCHEMICAL AND BIOPHYSICAL RESEARCH COMMUNICATIONS, ACADEMIC PRESS INC. ORLANDO, FL, US, vol. 176, no. 1, 15 April 1991 (1991-04-15), pages 406-412, XP000147128 ISSN: 0006-291X
- BONETTO V ET AL: "C-TERMINAL SEQUENCE ANALYSIS OF PEPTIDES AND PROTEINS USING CARBOXYPEPTIDASES AND MASS SPECTROMETRY AFTER DERIVATIZATION OF LYS AND CYS RESIDUES" ANALYTICAL CHEMISTRY, AMERICAN CHEMICAL SOCIETY. COLUMBUS, US, vol. 69, no. 7, 1 April 1997 (1997-04-01), pages 1315-1319, XP001097721 ISSN: 0003-2700

## Description

### BACKGROUND OF THE INVENTION

Over the last decade, polypeptide mapping experiments combining matrix-assisted laser desorption/ionization (MALDI) mass spectrometry (MS) with genomic database searching have proven to be a powerful tool for the identification of isolated proteins (*see*, Lahm HW et al., Electrophoresis 21:2105 (2000)). More recently, several groups have begun to explore the combination of various liquid separation methods followed by MALDI MS for the analysis of polypeptide mixtures of even greater complexity (*see,* Griffin TJ et al., Anal. Chem. 73:978 (2001); Preisler J et al., Anal. Chem. 72:4785 (2000); Johnson T et al., J. Anal. Chem. 73:1670 (2001); Riggs L et al., J. Chromatogr. A 924:359 (2001)). The "permanent record" obtained by deposition of a separation column's eluent onto a MALDI target plate provides several advantages compared to a real time coupling of the separation method and mass spectrometer in on-line electrospray ionization mass spectrometry (*see*, Griffin TJ et al., Anal. Chem. 73:978 (2001)). However, despite its apparent simplicity, the performance of MALDI MS can greatly be affected by competitive ionization effects, especially in complex mixtures. Operational variables such as the nature of the matrix, the pH, and the overall rate of crystallization can also effect performance (*see,* Cohen SL et al., Anal. Chem. 68:31 (1996)). In fact, the chemical properties of the amino acid side chains themselves have been found to effect a polypeptide's signal intensity (*see*, Kratzer R et al., Electrophoresis 19:1910 (1998)). Even if the suppression due to differing hydrophobicities is minimized by performing reversed-phase HPLC (*see*, Griffin TJ et al., Anal. Chem. 73:978 (2001); Riggs L et al., J. Chromatogr. A 924:359 (2001)) or unique crystallization methods *(see,* Preisler J et al., Anal. Chem. 72:4785 (2000)), other suppression effects remain.

Krause and coworkers investigated the dominance of arginine-containing polypeptides in the MALDI MS analysis of tryptic digests, and found that polypeptides containing an arginine residue exhibit a four- to eighteen-fold increase in signal intensity compared to those polypeptides containing a lysine (*see,* Krause E et al., Anal. Chem. 71:4160 (1999)). This bias is thought to result from the higher basicity of the arginine residue compared to lysine. Greater sequence coverages could in theory be obtained using a labeling methodology that either increases the basicity of lysine residues (*see,* Bonetto V et al., Anal. Chem. 69:1315 (1997); Brancia FL et al., Rapid Commun. Mass Spectrom. 14:2070 (2000); Hale JE et al., Anal. Biochem. 287:110 (2000); Keough T et al., Rapid Commun. Mass Spectrom. 14:2348 (2000); Beardsley RL et al., Rapid Commun. Mass Spectrom 14:2147 (2000)), or reduces the basicity of arginine residue (*see*, Cui H et al., J. Chromatogr. A 704:27 (1995)). However, the former method has attracted more attention, because reducing the basicity of arginine is considered detrimental to the overall sensitivity.

Based on earlier work done with proteins, several groups (Brancia FL et al., Rapid Commun. Mass Spectrom. 14:2070 (2000); Hale JE et al., Anal. Biochem. 287:110 (2000); Keough T et al., Rapid Commun. Mass Spectrom. 14:2348 (2000); Beardsley RL et al., Rapid Commun. Mass Spectrom. 14:2147 (2000)) have recently reported the use of *O-*methylisourea to convert lysine terminal residues in tryptic digests to more basic homoarginine residues. In all cases, this treatment resulted in higher sequence coverages in polypeptide mapping experiments compared to the underivatized tryptic digest. Although effective in leveling ionization efficiencies, this label does not perform other functions typically enabled through other derivatization methodologies. For example, differential quantitation experiments (*see*, Gygi SP et al., Nat. Biotechnol. 17:994 (1999)) would require the incorporation of the relatively expensive ¹³C and ¹⁵N stable isotopes into *O-*methylisourea, allowing a maximum mass difference of only 3 Da. Further, unlike other charge-localizing labels that affect the observed pattern in tandem MS experiments (*see*, Roth KDW et al., Mass Spectrometry Reviews 17:255 (1998)), *O*-methylisourea derivatized polypeptides provide only very limited additional sequence information compared to their unlabeled counterparts.

In view of the foregoing, what is needed in the art are new labeling reagents that are specific for lysine residues. Derivatizing agents are needed that increase the sequence coverage obtained in polypeptide mapping experiments. Moreover, labeling reagents are needed wherein stable isotopic enrichment synthesis is facile, thereby making quantitative differentiation studies easy to perform. The present invention fulfills these and other needs.

European Patent No. 0097031 discloses nonapeptide and decapeptide analogs of LHRH, including LHRH analogs having a modified Lys residue.

The technical publication by Levine et al In Anal. Biochem, 1997, Vol. 247, 83-88 discloses measurement of specific protease activity utilizing fluorescence polarization. The document also discloses peptides with modified Lys residues.

The technical publication by Rivier et al in Biochem Biophys. Res. Comm., 1991, Vol. 176, 406-412 also discloses LHRH analogs having a modified Lys residue.

The technical publication by Peters et al in Rapid.Comm.Mass.Spectr., 15 November, 2001, Vol 15, 2387-2392 also discloses peptides having modified Lys residues. However such document was published after the earliest priority date of the present invention.

### SUMMARY OF THE INVENTION

The present invention provides multifunctional labels for use in for example, proteomics studies. Advantageously, the labels of the present invention are both lysine specific and increase the overall sequence coverage obtained in polypeptide mapping experiments, by for example, increasing the ionization efficiencies of lysine-containing fragments. In certain aspects, the labels of the present invention can be used to measure differential quantitation, as for example, a stable isotope (*e*.*g*., deutcritun(s)) can easily be introduced during synthesis: In one aspect, a C-terminal derivatized lysine biases the fragment ion intensities strongly toward C-terminal fragment ions ("y-ions"), resulting in a highly simplified tandem mass spectrum. The additional elucidation of the number of lysine residues afforded by the labeling reaction during MS analysis enables more efficient protein identification by providing additional stringent criteria for database searching. In its extreme form, this compositional information can be combined with extremely accurate mass measurements to enable protein identification to be performed based solely on this information without any experiments to determine amino acid sequence information. Although applicable to ESI-based methodologies, this scheme is particularly effective when a laser desorption ionization based fractionation and subsequent analysis process is employed.

In one embodiment, the present invention provides a method for mass spectrometric polypeptide analysis, comprising:
a) ionizing a modified polypeptide having a lysine residue of Formula Ib as defined in claim 1; and
b) analyzing the results of the ionized modified polypeptide.

In a preferred embodiment, the present invention provides a method for analyzing a sequentially labeled polypeptide, comprising:

incubating the polypeptide with a first label to form a first labeled polypeptide;

incubating the first labeled polypeptide with a second label to form a second labeled polypeptide; wherein at least one of the first label or the second label is a compound having Formula IIa:

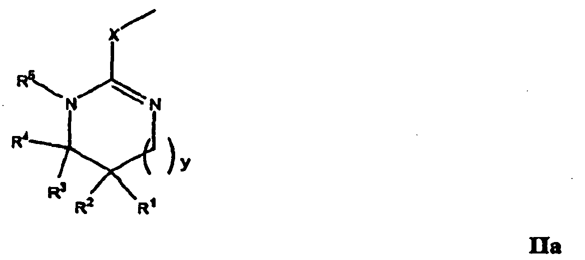

wherein R¹, R², R³ and R⁴ are each independently a member selected from the group consisting of hydrogen, deuterium, halogen, hydroxyl, alkyl, alkoxy, aryl, and an affinity tag; or, alternatively, R², R³ and the carbons to which they are attached join to form an n-membered carbocyclic, heterocyclic, aryl or heteroaryl ring;

*n* is 4 to 8;

R⁵ is a member selected from the group consisting of hydrogen, halogen, hydroxyl, alkyl, alkoxy, aryl and an affinity tag;

X is a heteroatom selected from the groups consisting of O and S; and y is 0 and analyzing the second labeled polypeptide with a mass spectrometer (*e*.*g*., high-mass accuracy).

In a preferred embodiment, the method further comprises labeling the second labeled polypeptide with a third label to form a third labeled polypeptide. The first, second and third labels are independently selected from a compound of Formula IIa, a cysteine labeling reagent, an arginine labeling reagent, a carboxylic acid labeling reagent, or other known specific amino acid or amino acid functional group labeling reagent.

In still yet another embodiment, the present invention provides a method for increasing the ionization efficiency of a lysine-containing polypeptide, comprising:

a) incubating a lysine-containing polypeptide with a compound of Formula IIa to form a modified polypeptide having a lysine residue of Formula Ib according of claim 12

These and other advantages and embodiments will become more apparent when read with the detailed description and drawings which follow.
The scope of the present invention is defined by the claims. Any subject matter disclosed herein that falls outside of the scope of the claims is included for information purposes only.

### BRIEF DESCRIPTION OF THE DRAWINGS

**Figure 1** illustrates a reaction of a lysine residue with a label of the present invention (*e*.*g*., 2-methoxy-4,5-dihydro-1*H*-imidazole) to form a compound of Formula I.

**Figure 2** illustrates a MALDI-FTICR spectra of equine myoglobin tryptic digest before (top) and after (bottom) reaction with a label of the present invention.

**Figure 3** illustrates various compounds of the present invention.

**Figure 4** illustrates a synthesis of a compound of Formula II.

**Figures 5** (A-C) illustrate (A) a tandem mass spectra of underivatized SIGSLAK, (B) its *O*-methylisourea derivative, and (C) after reaction with a label of the present invention. * indicates water loss peaks

**Figure 6** illustrates an isotopic cluster from a MALDI-FTICR spectrum of equine myoglobin tryptic polypeptides differentially labeled with one (left), two (center), or three (right) labels of the present invention.

**Figure 7** illustrates a sequential labeling method of the present invention.

**Figure 8** illustrates sequential site selective labeling of peptides.

**Figure 9** illustrates a comparative example.

### DETAILED DESCRIPTION OF THE INVENTION

### A. DEFINITIONS

As used herein, the term "alkyl" denotes branched or unbranched hydrocarbon chains, preferably having about 1 to about 8 carbons, such as, methyl, ethyl, *n*-propyl, *iso*propyl, *n*-butyl, *sec*-butyl, *iso*-butyl, *tert*-butyl, octa-decyl and 2-methylpentyl. These groups can be optionally substituted with one or more functional groups which are attached commonly to such chains, such as, hydroxyl, bromo, fluoro, chloro, iodo, mercapto or thio, cyano, alkylthio, heterocyclyl, aryl, heteroaryl, carboxyl, carbalkoyl, alkyl, alkenyl, nitro, amino, alkoxyl, amido, and the like to form alkyl groups such as trifluoro methyl, 3-hydroxyhexyl, 2-carboxypropyl, 2-fluoroethyl, carboxymethyl, cyanobutyl and the like.

"Alkylcarbamoyl" means an alkyl-NH-CO- group wherein alkyl group is defined herein. Preferred alkylcarbamoyl groups are those wherein the alkyl group is lower alkyl.

The term "alkoxy" denotes -OR, wherein R is alkyl.

"Alkoxycarbonyl" means an ester group; *i*.*e*., an alkyl-O-CO- group wherein alkyl is as defined herein. Representative alkoxycarbonyl groups include methoxycarbonyl, ethoxycarbonyl, t-butyloxycarbonyl, and the like.

The term "aryl" denotes a chain of carbon atoms which form at least one aromatic ring having preferably between about 6-14 carbon atoms, such as phenyl, naphthyl, and the like, and which may be substituted with one or more functional groups which are attached commonly to such chains, such as hydroxyl, bromo, fluoro, chloro, iodo, mercapto or thio, cyano, cyanoamido, alkylthio, heterocycle, aryl, heteroaryl, carboxyl, carbalkoyl, alkyl, alkenyl, nitro, amino, alkoxyl, amido, and the like to form aryl groups such as biphenyl, iodobiphenyl, methoxybiphenyl, anthryl, bromophenyl, iodophenyl, chlorophenyl, hydroxyphenyl, methoxyphenyl, formylphenyl, acetylphenyl, trifluoromethylthiophenyl, trifluoromethoxyphenyl, alkylthiophenyl, trialkylammoniumphenyl, amidophenyl, thiazolylphenyl, oxazolylphenyl, imidazolylphenyl, imidazolylmethylphenyl, and the like.

"Arylcarbamoyl" means an aryl-NHCO- group, wherein aryl is defined herein.

"Aryloxy" denotes -OR, wherein R is aryl.

"Aryloxycarbonyl" means an ester group; *i*.*e*., an aryl-O-CO- group wherein aryl is as defined herein. Representative aryloxycarbonyl groups include phenoxycarbonyl, and the like.

As used herein a leaving group, LG, is an atom (or a group of atoms) that is displaced as a stable species taking with it the bonding electrons. Typically, the leaving group is an anion (*e*.*g*. C1-) or a neutral molecule (*e*.*g*. H₂O). The better the leaving group, the more likely it is to depart. A "good" leaving group can be recognized as being the conjugate base of a strong acid. Suitable leaving groups include, but are not limited to, a halogen, an alkoxy group, an alkylthio group, an aryloxy group, a tosyl group and an arylthio group. Those of skill in the art will know of other leaving groups suitable for use in the present invention.

As used herein, the terms "polypeptide", "peptide" and "protein" are used interchangeably to include a molecular chain of amino acids linked through peptide bonds. As used herein, the terms do not refer to a specific length of the product. Thus, "peptides," "oligopeptides," and "proteins" are included within the definition of polypeptide. As used herein, the terms include post-translational modifications of the polypeptide, for example, glycosylations, acetylations, phosphorylations and the like. In addition, protein fragments, analogs, mutated or variant proteins, fusion proteins and the like are included within the meaning of polypeptide.

Certain compounds of the present invention possess asymmetric carbon atoms (optical centers) or double bonds; the racemates, diastereomers, geometric isomers and individual isomers are all intended to be encompassed within the scope of the present invention.

The compounds of the present invention can also contain unnatural proportions of atomic isotopes at one or more of the atoms that constitute such compounds. For example, the compounds can be isotopically labeled with stable isotopes, such as for example deuterium (²H), nitrogen-15 (¹⁵N), carbon-13 (¹³C) and combinations thereof. All isotopic variations of the compounds of the present invention, whether stable or not, are intended to be encompassed within the scope of the present invention.

The compounds and methods of the present invention have widespread utility. Applications include, but are not limited to, facilitation of biological studies requiring rapid determination of peptide or polypeptide sequences; identification of post-translational modifications in proteins and for the identification of amino acid modifications in variant proteins such as those used in, for example, drug discovery; aiding the design of oligonucleofide probes for gene cloning; rapid characterization of products formed in directed evolution studies; combinatorial chemistry and peptide library identification; and proteomics.

### B. MODIFTED POLYPEPTIDES

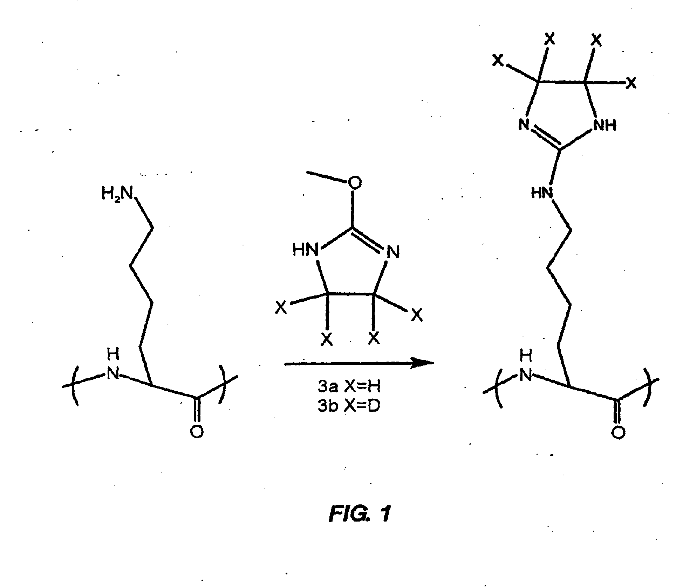

Figure 1 shows the reaction for the conversion of lysine residues to their imidazol-2yl counterparts. In certain aspects, the labeling reagents of the present invention are highly water soluble, and can directly be added to the polypeptide solution of interest. In certain aspects, the label itself serves as a base to accelerate the rate of reaction. The resulting chemical moiety is similar in structure to a guanidinium group. Advantageously, the labels of the present invention increase the ionization efficiency of lysine-containing polypeptide(s), thus enabling a myriad of applications, such as increasing the level of sequence coverage observed in polypeptide mapping experiments. In fact, the additional elucidation of the number of lysine residues afforded by the present labels and methods enables more efficient protein identification.

The present invention provides compounds and methods wherein individual polypeptides having a lysine at for example, the C-terminus (*e*.*g*., tryptic digests), as well as internal lysine residues, are reacted with a compound of Formula II (*e.g.,* 2-methoxy-4,5-dihydro-1*H*-imidazole), converting the lysine residue(s) to their corresponding inventive derivative(s) (*e.g.,* 4,5-dihydro-1*H*-imidazol-2-yl). Advantageously, laser-desorption ionization mass spectra (*e*.*g*., MALDI) of derivatized digests exhibit a greater number of more intense features than their underivatized counterparts, thus increasing the information obtained in for example, polypeptide mapping experiments. Additionally, MS/MS spectra of the derivatized polypeptides are greatly simplified in comparison to their native species, yielding primarily an easily interpretable series of y-ions. Those of skill in the art will know of other techniques of operations, such that for example, the number of tandem mass spectrometric steps are reduced and simplified using the present compositions and methods. In certain embodiments, the present invention provides a polypeptide having a modified lysine residue of Formula I:
In certain preferred embodiments, the polypeptide of Formula I has Formula Ib: 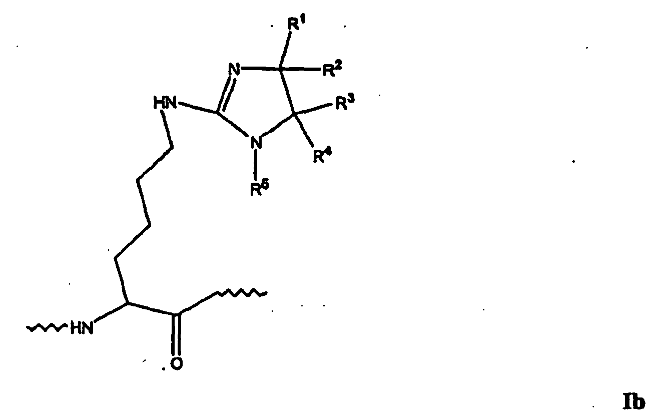 wherein R¹, R², R³ R⁴, R⁵, and n have been defined in the claims.

In certain aspects, the present invention provides labels that increase the ionization efficiencies of lysine containing tryptic peptides. Increasing ionization efficiencies of lysine terminated tryptic peptides is particularly advantageous in laser desorption methods such as MALDI-based experiments. Advantageously, by increasing the ionization efficiencies of lysine signals, these lysine signals will not be overwhelmed by, for example, signals from arginine containing peptides such as those prevalent in MALDI-based analyses.

In certain aspects, the labels of the present invention are useful for laser desorption ionization MS techniques. These techniques include, but are not limited to, MALDI, IR-MALDI, UV-MALDI, liquid-MALDI, surface-enhanced LDI (SELDI), surface enhanced neat desorption (SEND), desorption/ionization of silicon (DIOS), laser desorption laser ionization MS, laser desorption/two-step laser ionization MS, and the like. Those of skill in the art will know of other ionization techniques as well as other mass spectrometric techniques useful in the present methods. (*see, for example*, Merchant M, Weinberger SR Electrophoresis 21, (6): 1164-1177 2000; Issaq HJ, Veenstra TD, Conrads TP, et al. Biochem Bioph Res Co 292 (3): 587-592, 2002, Thomas JJ, Shen ZX, Crowell JE, et al. P Natl Acad Sci, 98 (9): 4932-4937, 2001 and Kruse RA, Rubakhin SS, Romanova EV, et ai. J. Mass Spectrum, 36 (12): 1317-1322, 2001).

Moreover, although the label is useful in laser-desorption methods (*e*.*g*., MALDI), it is also completely compatible with electrospray ionization (ESI). Advantageously, the label does not interfere in the tandem MS pattern of multiply charged, singly labeled ions typical in ESL In addition, in certain aspects, the labels of the present invention affect tandem MS breakdown in for example, +1 charged species. The resulting tandem MS patterns are useful in identifying unknown analytes, especially proteins and peptides.

As described above, various mass spectrometric techniques are used to ionize derivatized polypeptides of the present invention. In operation, mass spectrometry separates the ions according to their mass to charge ratio (m/z). Tandem mass spectrometers operate by using this separation of ions as a first fractionation step. Before entering the second mass spectrometer, ion fractions from the first are fragmented (*e.g.*, collisionally dissociated by passage through a neutral gas, to induce fragmentation). These fragments exist as a family of subset ions of the original parent ions. Analysis of the m/z spectrum of these subset ions are used to determine the parent polypeptide (or protein).

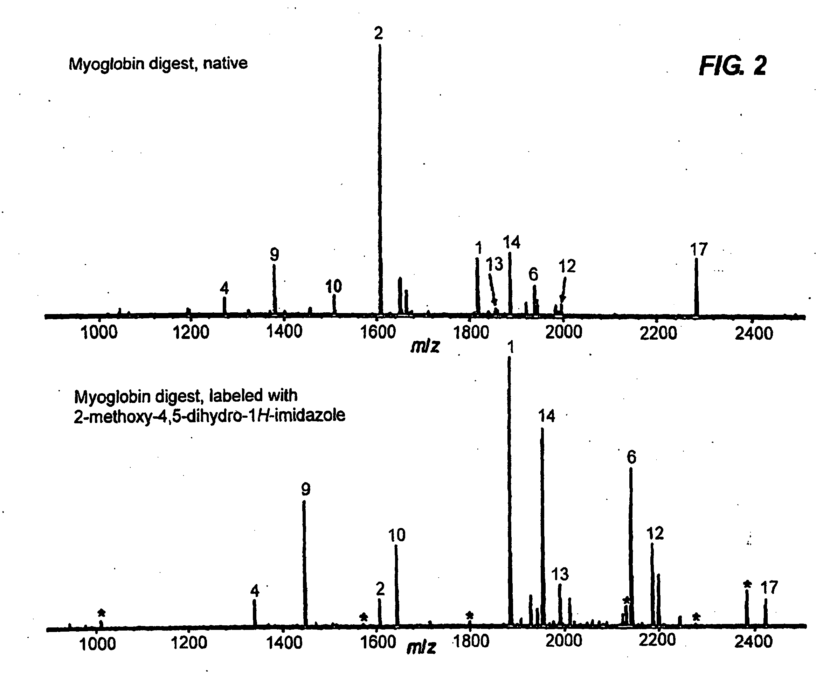

Figure 2 shows an illustrative MADLI mass spectra of a tryptic digest of equine myoglobin both before and after reaction with a compound of the present invention (*e.g.*, 2-methoxy-4,5-dihydro-1H-imidazole). The spectrum of the native digest shows 10 polypeptides covering a total of 166 amino acids (75.8% sequence coverage), whereas the spectrum of the labeled digest exhibits 18 polypeptides covering a length of 309 amino acids (96.1%). Thus, the labels and methods of the present invention significantly increase the overall sequence coverage and nearly double the amount of redundant information obtained.

Table 1 tabulates a summary of tryptic polypeptide masses of equine myoglobin detected with MALDI- FTICRMS before and after reaction with a label of the present invention (*e*.*g*., 2-methoxy-4,5-dihydro-1*H*-imidazole). Table 1 further details the peak assignments in both spectra of Figure 2. As shown therein, non-lysine containing polypeptides exhibit no increase in mass after the derivatization procedure (#2). Lysine-containing polypeptides increase in mass by 68 Da, or multiples thereof, depending on the total number of lysines present. Despite the fact that the majority of polypeptides found contain from one to three missed cleavages, partially derivatized species are nearly nonexistent. This demonstrates both the selectivity as well as the reactivity of the label. The largest peak by far in the native digest is the arginine-terminated polypeptide (#2). By contrast, the intensity of this peak is dramatically decreased in the labeled digest, whereas nearly every labelled lysine-containing polypeptide exhibits significantly higher relative intensity.

**Table 1**

| | | | Experimental Data from MALDI-FTICRMS | | |
|---|---|---|---|---|---|
| Polypeptide # | Sequence | # of Lysine Miscleavages | Native Detected Mass | Labeled Detected Mass | # of Labels |
| 1 | GLSDGEWQQVLNVWGK | 0 | 1814.90 | 1882.91 | 1 |
| 2 | VEADIAGHGQEVLIR | 0 | 1605.84 | 1605.84 | 0 |
| 3 | GLSDGEWQQVLNVWGKV- | 1 | | 3470.69 | 1 |
| | EADIAGHGQEVLIR | | | | |
| 4 | LFTGHPETLEK | 0 | 1270.66 | 1338.70 | 1 |
| 5 | LFTGHPETLEKFDK | 1 | | 1796.94 | 2 |
| 6 | LFTGHPETLEKFDKFK | 2 | 1936.01 | 2140.11 | 3 |
| 7 | FKHLKTEAEMKASEDLK | 3 | | 2276.18 | 4 |
| 8 | HLKTEAEMKASEDLKK | 3 | | 2129.11 | 4 |
| 9 | HGTVVLTALGGILK | 0 | 1377.83 | 1445.86 | 1 |
| 10 | HGTVVLTALGGILKK | 1 | 1505.94 | 1642.00 | 2 |
| 11 | KKGHHEAELKPLAQSHATK | 3 | | 2381.31 | 4 |
| 12 | KGHHEAELKPLAQSHATK | 2 | 1981.06 | 2185.15 | 3 |
| 13 | GHHEAELKPLAQSHATK | 1 | 1852.97 | 1989.02 | 2 |
| 14 | YLEFISDAIIHVLHSK | 0 | 1884.03 | 1952.02 | 1 |
| 15 | HPGDFGADAQGAMTK | 0 | | 1569.72 | 1 |
| 16 | YLEFISDAIIHVLHSKHPG- | 1 | | 3503.72 | 2 |
| | DFGADAQGAMTK | | | | |
| 17 | ALELFRNDIAAKYKELGFQG | 1 | 2282.21 | 2419.29 | 2 |
| 18 | YKELGFQG | 1 | | 1008.51 | 1 |

As such, using the compounds and methods of the present invention it is possible to determine the number of lysine residues in a polypeptide fragment or parent protein. The additional elucidation of the number of lysine residues afforded by the present labels and methods enables more efficient protein identification. As explained more fully below, by comparing the empirically obtained mass to members of a database of theoretical masses for a plurality of *in silico* proteolytic peptides wherein a match is obtained, the resultant match is indicative of the presence of the matched peptide in the sample.

### C. COMPOUNDS

In one embodiment, the present invention provides a compound of Formula 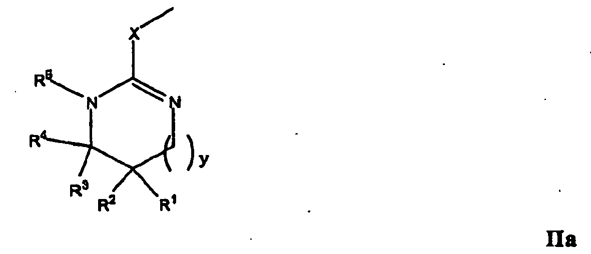 wherein R¹, R², R³, R⁴, R⁵, y, n, and X have been described.

In certain other preferred aspects, the compound has Formula IIb 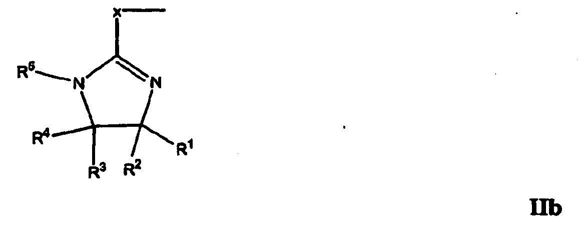 wherein R¹ R², R³, R⁴, R⁵, n, and X have been described.

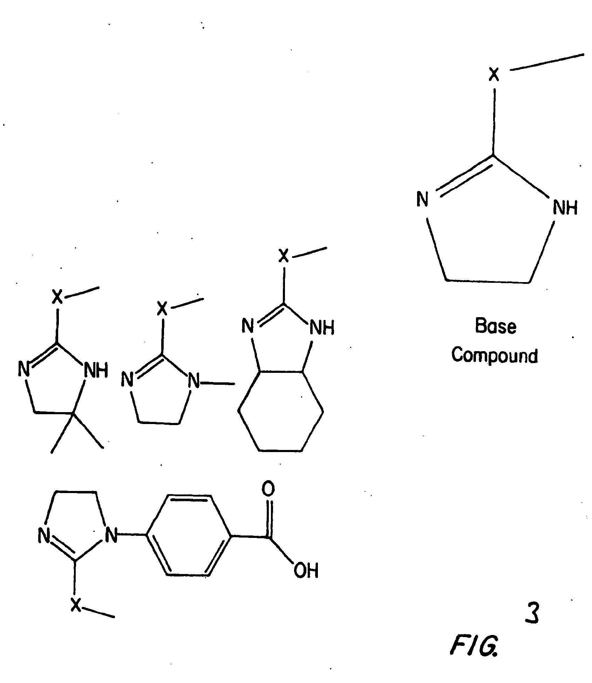

Figure 3 set forth preferred embodiments, In one embodiment, compounds comprise an affinity tag. An affinity tag is one member of a complementary binding pair. Exemplary binding pairs include any haptenic or antigenic compound in combination with a corresponding antibody or binding portion or fragment thereof (*e*.*g*., digoxigenin and anti-digoxigenin; fluorescein and anti-fluorescein; dinitrophenol and anti-dinitrophenol; bromodeoxyuridine and anti-bromodeoxyuridine; mouse immunoglobulin and goat anti-mouse immunoglobulin) and nonimmunological binding pairs (*e.g.,* biotin-avidin, biotin-streptavidin, hormone (*e.g.,* thyroxine and cortisol)-hormone binding protein, receptor-receptor agonist or antagonist (*e.g.,* acetylcholine receptor-acetylcholine or an analog thereof), IgG-protein A, lectin-carbohydrate, enzyme-enzyme cofactor, enzyme-enzyme-inhibitor, and complementary polynucleotide pairs capable of forming nucleic acid duplexes) and the like.

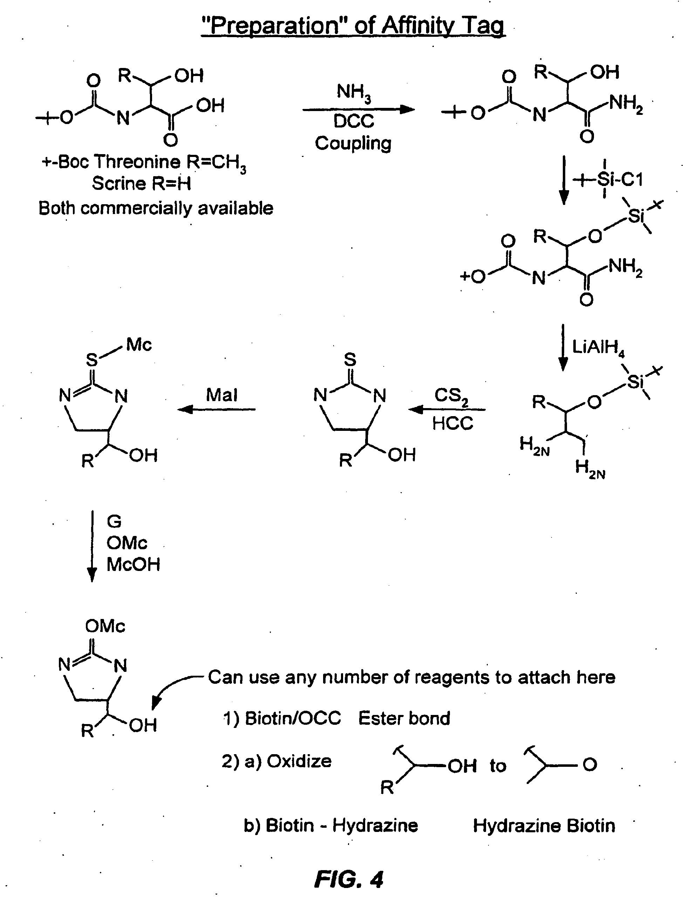

Figure 4 shows an exemplary method of synthesizing a compound having an affinity tag. In this embodiment, R³ is an optionally substituted alkyl group having a free hydroxyl group. As shown in Figure 4, the free hydroxyl is derivatized with a biotin functionality using methods known in the art. The affinity tag comprising biotin can then be used to modify a polypeptide containing lysine. The biotinylated affinity tag can be separated using for example, an affinity column having streptavidin.

### D. SIMPLIFIED TANDEM MASS SPECTRA

In other aspects, the present invention provides methods of simplifying mass spectra such as tandem mass spectra, by using the compounds of the present invention. In an illustrative example, not intending to be limiting, several model polypeptides containing lysine at their C-terminus were labeled with both *O*-methylisourea (comparative) as well as 2-methoxy-4,5-dihydro-1*H*-imidazole (inventive) and their tandem mass spectra along with those of the native polypeptides were obtained using an ion trap MS.

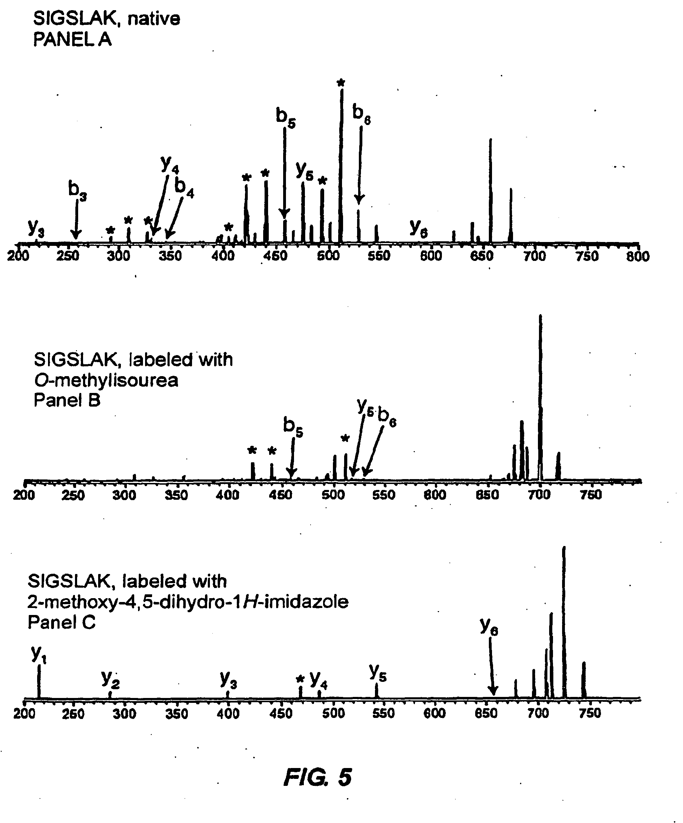

Figure 5 shows the spectra for the heptapeptide SIGSLAK. The native polypeptide (top spectrum) shows both a series of N-terminal fragment ions and C-terminal fragment ions ("b-and y-ions, respectively"), but is complicated by a number of intense peaks assigned as losses of water (marked with *). In the comparative spectrum, the polypeptide labeled with *O*-methylisourea (middle spectrum) exhibits some of these same peaks, but offers no new sequence information. In stark contrast, the fragmentation pattern of the polypeptide labeled with an inventive compound 2-methoxy-4,5-dihydro-1H-imidazole (bottom spectrum), is greatly simplified compared to that of the native polypeptide, containing mostly easily interpretable series of y-ions. Each peak is shifted 68 Da higher than the y-ion series of the native polypeptide (or 72 Da if using the D4 version of the label) due to the presence of the derivatized lysine residue, that is now completely distinct in mass from glutamine residues. Other polypeptides investigated (VAITVLVK, YGGFLK, VQGEESNDK) exhibited the same proclivity for the near exclusive formation of y-ions, establishing that this label is also valuable for obtaining sequence information.

As will be appreciated by one of skill in the art, tandem MS patterns of+1 charge state peptides containing C-terminal lysines (*e.g.,* as generated by trypsin) are significantly simpler in the labeled form than the unlabeled form. This occurs regardless of whether those ions are formed by MALDI or ESI. However, ESI methods typically produce multiply charged ions vs. MALDI methods that primarily produce singly charged species.

In terms of the label's performance with typical tryptic multiply-charged ions in ESI, the following is an illustrative embodiment, not intending to be limiting. A myoglobin tryptic digest was divided into three parts. The first part was kept as is, the second part was labeled with the compound of this invention, and the third part was first labeled with the compound of the invention, and then subsequently labeled with the N-hydroxysuccinimide ester of acetic acid to label the *N*-termini of the all the tryptic peptides. The tandem MS patterns of the +2 charge state of peptide #9 in Table 1 for each sample were obtained by µHPLC MS/MS using an ESI interface and compared. When labeled with the compound of the present invention, the tandem MS pattern of the labeled peptide remained essentially unchanged from that of the native peptide, except that all the members of the y-ion series showed a mass shift of +68Da. Subsequent labeling of the *N*-terminus of the peptide as described above resulted in an additional shift of all the b-ion species by +42Da. Thus, the chemical selectivity afforded by the present labels and methods enables the facile assignment and interpretation of tandem MS fragmentation patterns.

Although the labels and methods of the present invention are applicable to MALDI applications, they are also very useful for non-MALDI based applications as well. Advantages in ESI applications, include, but are not limited to, the ability to effect differential quantitation, the ability to determine the number of lysines, and all its advantages, the ability to aid in interpreting tandem MS patterns, and the like.

### E. DIFFERENTIAL QUANTITATION

The present invention provides methods for differential quantitation using the compounds and derivatives disclosed herein. In certain aspects, samples to be compared are reacted with different isotopic versions of the same reagent, and the two derivatized samples are combined. The result is a series of isotopically labeled polypeptide pairs, with the relative concentration of each member of a given pair being directly proportional to its signal intensity. Advantageously, the methods of the present invention provide differential quantitation while simultaneously maintaining the label's other desirable properties.
Methods of differential quantitation are disclosed in for example, Gygi SP et al., Nat. Biotechnol. 17:994 (1999); Munchbach M et al., Anal. Chem. 72:4047 (2000); Ji J et al., J. Chromatogr. B 745:197 (2000); Goshe MB et al., Anal. Chem. 73:2578 (2001); Oda Y et al., Nat. Biotech. 19:379 (2001); Zhou HL et al., Nat. Biotech. 19:375 (2001); Goodlett DR et al., Anal. Chem. 72:1112 (2000) each of which are incorporated herein by reference in their entirety for all purposes.

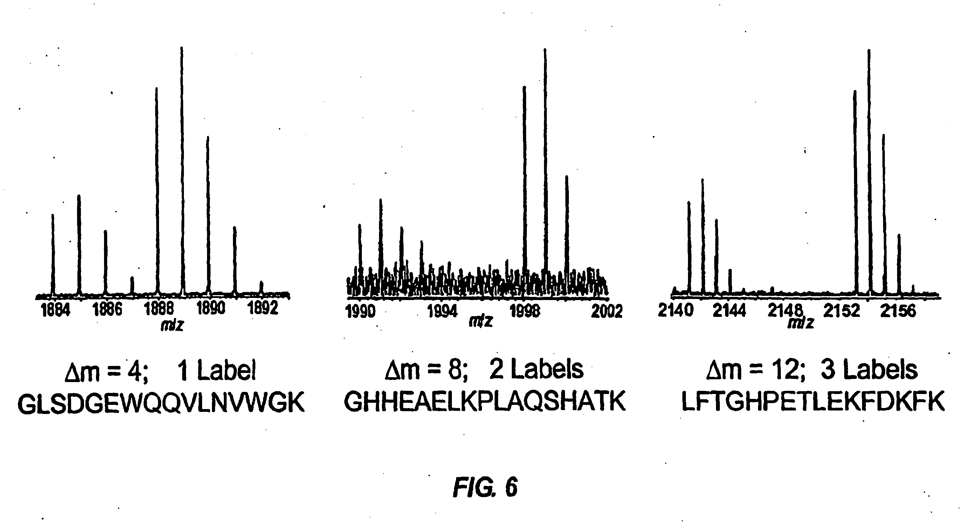

In one embodiment, the present invention provides lysine specific labels which enable the determination of the number of lysine(s) in a peptide derivative by being produced in at least two isotopic forms. Figure 6 shows differential quantitation using compounds and methods of the present invention. In an illustrative embodiment, not in any way intending to be limiting, an equine myoglobin tryptic digest was derivatized separately with two isotopic versions (*e*.*g*., the d0 and d4 forms) of 2-methoxy-4,5-dihydro-1H-imidazole, and recombined in a ratio of 1:3 respectively. As shown therein, the ratio of the two species seen in each MALDI-FTICR mass spectrum reflects the stoichiometry of the original mixture. The left spectrum shows an isotopic pair differing in mass by 4 Da, indicating the presence of a single label. After accounting for the additional mass due to the labeling reaction, this polypeptide is identified as polypeptide #1 in Table 1, possessing a single lysine residue. Similarly, the center and right spectra display isotopic clusters differing in mass by 8 and 12 Da. After accounting for the added mass of the labels, these polypeptides are identified as #13 and #6, possessing two and three lysine residues, respectively. In addition to affecting differential quantitation, the additional elucidation of the number of lysine residues afforded by the present labels and methods enables more efficient protein identification.

As such, in certain embodiments, the present invention provides a method for performing differential quantitation of a lysine-containing polypeptide, the method comprising:

a) incubating a first sample of a lysine-containing polypeptide with a first isotopic version of a compound of Formula IIa to form a first modified polypeptide having a lysine residue of Formula Ib.

b) incubating a second sample of the lysine-containing polypeptide with a second isotopic version of a compound of Formula IIa to form a second modified polypeptide having a lysine residue of Formula Ib

c) combining the first sample and the second sample of the modified polypeptide having a lysine residue of Formula Ib to form a mixture; and

d) ionizing the mixture to form a series of isotopically labeled polypeptide pairs, wherein the relative concentrations of the first modified polypeptide having a lysine residue of Formula I and the second modified polypeptide having a lysine residue of Formula I are directly proportional to their relative signal intensities, and wherein the mass difference between the two modified polypeptides indicates the number of lysine(s) that are present in the lysine-containing polypeptide.

The present invention is not limited to isotopic display. For example, rather than labeling two different samples each with a different isotopic version and recombining the samples, a single sample can be labeled with specific amounts (*e.g.,* equimolar amounts) of at least two isotopic labels, wherein the mass difference between the at least two isotopic clusters arising from the peptide/isotope mass difference per label indicate the number of lysine(s) that are present. As the present invention provides lysine specific labels which enable the determination of the number of lysine(s) in a peptide, the identification of a protein from its peptides is readily obtained.

As such, in certain aspects, the present invention provides a method for determining the number of lysine(s) in a lysine-containing polypeptide, comprising:

a) incubating a sample of a lysine-containing polypeptide with a first isotopic version of a compound of Formula IIa and a second isotopic version of a compound of Formula IIa to form a mixture having a first modified polypeptide having a lysine residue of Formula Ib and a second modified polypeptide having a lysine residue of Formula Ib,

b) ionizing the mixture to form a series of isotopically labeled polypeptide pairs, wherein the mass difference between the first modified polypeptide having a lysine residue of Formula I and the second modified polypeptide having a lysine residue of Formula I indicates the number of lysine(s) that are present in the lysine-containing polypeptide.

Polypeptide or peptides may be produced by any means. For example, if necessary, the polypeptide of interest is isolated for analysis. Several procedures may be utilized for isolation including, for example, one-dimensional and two-dimensional gel electrophoresis. As another example, polypeptides may be synthesized through combinatorial chemistry methods well known in the art.

Digestion may occur through any number of methods, including in-gel or on a membrane, preferably in-gel. See, *e*.*g*., Shevchenko et al., "Mass Spectrometric Sequencing of Proteins from Silver-Stained Polyacrylamide Gels", Analytical Chemistry, Vol. 68, pp. 850-858 (1996). However, it is possible to digest the polypeptide either enzymatically or chemically, preferably enzymatically. It is most preferable to utilize a digestion procedure which yields a basic or hydrophobic residue, most preferably basic, at or near the C-terminus of the resulting peptides.

While many methods may be utilized for this procedure, it is preferred to enzymatically digest the polypeptide using, for example, trypsin, endoproteinase Lys C, endoproteinase Arg C, or chymotrypsin, preferably, trypsin, endoproteinase Lys C, or endoproteinase Arg C, and most preferably trypsin. Trypsin, endoproteinase Lys C, and endoproteinase Arg C are preferable because the resulting peptides of the polypeptide will typically terminate at the C-terminus with an arginine or lysine residue (basic residues), with the exception, of course, of the original C-terminus of the polypeptide. Other enzymes are also suitable, especially if basic residues occur at or near the C-terminus of the resulting peptides. Chymotrypsin is also preferred for digestion, which typically cleaves at hydrophobic amino acid residues. Chemical digestion is also useful. For example, digestion with cyanogen bromide is useful.

However, digestion is not always necessary, particularly when sequencing (but certainly not limited to) small polypeptides. In certain aspects, polypeptides include those having preferably less than about fifty amino acid residues, more preferably less than about forty residues, even more preferably less than about thirty residues, still more preferably less than about twenty residues, and most preferably less than about ten amino acid residues. For example, polypeptides may be characterized which are synthesized by well-known means, including combinatorial chemistry methods (a "synthetic polypeptide"). In this instance, it is most preferable to synthesize a polypeptide having basic or hydrophobic residue, preferably basic (most preferably arginine, homoarginine or lysine), at or near the C-terminus of the resulting polypeptide.

In other aspects, the protein or peptide sample is divided into a first portion and a second portion. The labels of the present invention can be used to derivatize the first portion of the sample with a first isotopic form, whereas the second portion of the sample is derivatized with a second isotopic form of the agent. Exemplary isotopes for use in the labels of the present invention include, but are not limited to, ²H, ¹³C, ¹⁴C, ¹⁵N, ¹⁸O, ³⁵Cl, ³⁷Cl, ⁷⁹Br, ⁸¹Br and combinations thereof.

As discussed above, the label is typically provided in two isotopic forms, in order to facilitate identification of the derivatized polypeptides. The sample proteins are contacted with the different isotopic versions of the same reagent (either in separate reactions or in a single pooled reaction). The result is a series of isotopically labeled polypeptide pairs, with the relative amount of each member of a given pair being directly proportional to the specific amounts (*e*.*g*., equimolar amounts) of the two isotopic labels. For example, using the inventive lysine-specific reagent 2-methoxy-4,5-dihydro-1H-imidazole in two isotopic forms, *e*.*g*., a deuterated version and a non-deuterated version. The derivatized proteins will be present in a mixture of deuterated and non-deuterated forms based on the number of lysines in the polypeptide.

While deuteration is a common isotopic form for use in the compounds methods of the present invention, stable isotopes of other atoms are optionally employed. For example, bromine is naturally present as a 50:50 ratio of ⁷⁹Br and ⁸¹Br; thus, bromine-labeled derivatizing agents inherently comprise a mixture of the two isotopes. While radioactive- labeled compounds are not commonly examined by MS, these labels can also be employed in the compounds and methods of the present invention.

In some embodiments, the isotopic forms are provided in "natural" proportions, for example, when using bromine-labeled agents. In other embodiments, the derivatizing agents comprise unnatural isotopic proportions of one or more stable isotopes, which can be selected or adjusted depending upon the experiment performed. Any isotopic variations of the derivatizing agents can be used the present invention, whether stable or not, and are intended to be encompassed within the scope of the present invention. Optionally, two or more isotopic forms of the derivatizing agent can be used in the methods of the present invention, with the appropriate adjustments made for the analysis of the resulting multiple products.

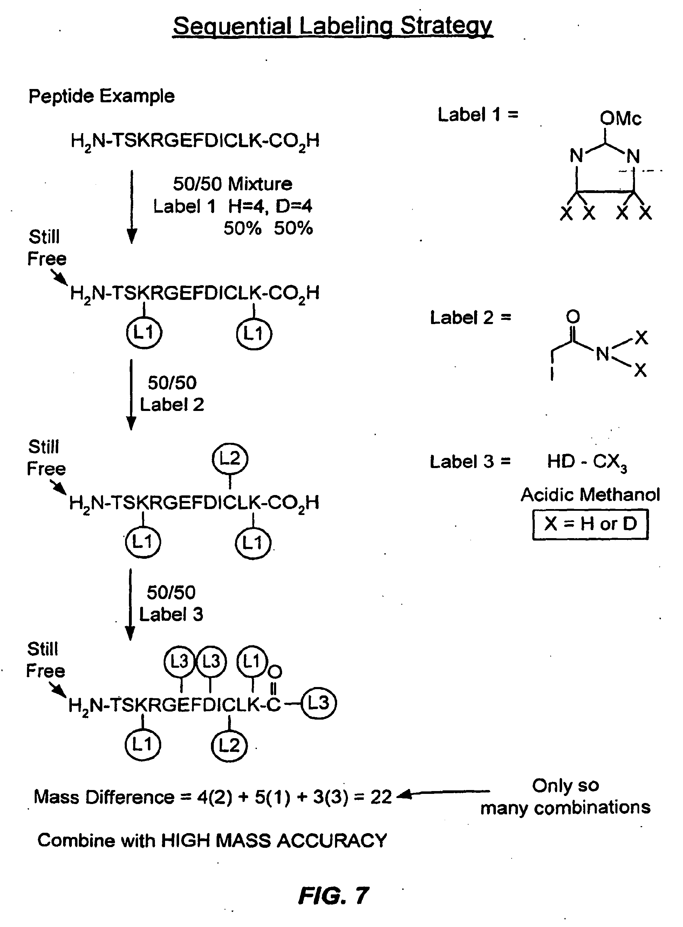

In one embodiment, the compounds and methods of the present invention can be used in conjunction with other labeling reagents (*e*.*g*., isotope coded affinity tags reagents (ICATs) and other methods which are specific for a particular amino acid (*see*, Griffin TJ *et al*., *supra*) in a sequential labeling strategy. Figure 7 is an illustrative embodiment, not in any way intending to be limiting. As shown therein, polypeptides are first labeled with a compound of Formula II to generate a polypeptide of Formula I. Thereafter, the polypeptide of Formula I can be labeled with another label such as an ICAT reagents specific for cysteinyl residues. In general, these ICAT reagents generally have three components: 1) a thiol-reactive group that reacts specifically to cysteine; 2) a polyether linker that can be synthesized as isotopically normal or heavy; and 3) an affinity tag such as biotin that allows the tagged polypeptides to be purified.

Similar to above, polypeptides in two samples are separately labeled on the side chains of their reduced cysteinyl residues using one of two isotopically different, but chemically identical sulfydryl-reactive reagents. One reagent is isotopically light d(0) and the other reagent is heavy d(8). The labeled protein mixtures are combined and enzymatically digested and thereafter, the labeled polypeptides can be isolated by affinity chromatography. In certain aspects, an affinity tag (*e*.*g*., biotin) is part of the ICAT reagent.
As the pairs of the polypeptide labeled with the d(0) and d(8) versions of the ICAT reagent are chemically identical, they serve as internal standards for polypeptide identification.

In addition to the first and second labels (*e*.*g*., a compound of Formula II and an ICAT reagent) set forth above, the compounds and methods of the present invention can be used with yet another label such as differential stable isotopic esterification of carboxylic acids, to provide for quantitation of polypeptides (*see*, Goodlett DR *et al. supra*). In this aspect, polypeptides from two samples are esterified with either d(0) or d(3) methanol (label 3 in Figure 7); the two samples are then mixed and analyzed as discussed previously.

Other cysteine-reactive compounds, include for example, Michael acceptors such as maleimide, acid halides, and benzyl halides. The maleimide-type labels are unique Michael acceptors for cysteine. Structurally, these reagents are ring compounds having an R group attached, allowing for multiple isotope substitution possibilities. One exemplary maleimide-based derivatizing agent is N-ethyl maleimide.

The ability of the free sulfhydryl group to form disulfide bonds offers another approach ability to label cysteine-containing proteins The free sulfhydryl of the cysteine residue can be reacted with a disulfide of a derivatizing agent, such that the interaction is converted to a disulfide bond. This reaction is reversible, and can be used to regenerate the original sulfhydryl group. Hundreds of derivatizing agents fall under this category and are available for use by one of skill in the art.

Finally, cysteine residues can be labeled using 4-vinylpyridine, as described in, for example, Ji et al. (2000) "Strategy for qualitative and quantitative analysis in proteomics based on signature peptides" J. Chromatography B 745:197-210.

Additional derivatizing agents include reagents that label carboxyl groups (such as carbodiimides, epoxides, diazoalkanes, diazoacetates, and esterification using methanolic HCl), histidine imidazole groups (diethylpyrocarbonate), and tyrosine side chains (N-acetylimidazole, tetranitromethane). Thus, potentially any derivatizing agents known or designed by one of skill in the art can be used in the methods of the present invention.

In certain aspects, the sample is divided into two (or more) portions. A first portion of the sample is contacted with the first isotopic form of the derivatizing agent, the second portion of the sample is contacted with the second isotopic form of the agent, etc.
Once labeled, the sample portions are recombined prior to further analysis. In an alternative embodiment, the isotopic forms of the derivatizing agent are provided as a mixture prior to contacting the sample (for example, as with the case of bromide-labeled compositions).

Furthermore, the labeling of the sample proteins via the derivatizing agent can be performed at any time prior to ionization of the sample fractions. Optionally, the sample and the derivatizing agent are contacted prior to fractionation, although derivatization could also be performed upon the eluted fractions. Furthermore, the derivatizing agent can be reacted with the sample either prior to or after the optional cleaving of the sample, as described below.

In certain instances, the compounds of the present invention can be used as probes to assess the proximity or distance between two different binding sites. For example, in one embodiment, a compound of the present invention comprises an affinity tag, a functional group capable of covalent attachment (*e*.*g*., iodoacetamide) or some other substance such as a quantum dot, a small molecule, a cell, a drug, or a liposome. As described above, an affinity tag is one member of a complementary binding pair (*e*.*g*., receptor and receptor agonist or antagonist). The affinity tag or functional group capable of covalent attachment anchors the compound to a target protein. For example, the affinity tag such as a receptor agonist can bind to its receptor. If a lysine residue is within proximity to the target protein, the compound of the present invention will also bind. Using this strategy, it is possible to take advantage of the anchor to assess the proximity of lysine residue close to the target protein binding site. It is therefore possible to assess whether certain amino acids are important for binding or for example, within the active site.

In certain other aspects, the compounds can be used in assay methods useful in the detection of a wide range of analyte species. The assay methods of the present invention can be performed in homogenous or heterogeneous formats. The methods are suitably used in numerous assay categories, including, but are not limited to, competitive assays, noncompetitive assays, sandwich assays and antibody assays.

In other aspects, the compound of the present invention further comprise a fluorophore such as a luminescence donor capable of luminescent emission. A wide range of luminescence donors are suitable for use in the present invention. In one embodiment, luminescence donors include organic fluorescence donors and nanoparticles. Suitable organic fluorescent donors include, but are not limited to, fluorescein, 5-carboxyfluorescein (FAM), rhodamine, 5-(2'-aminoethyl) aminonapthalene-1-sulfonic acid (EDANS), anthranilamide, coumarin, terbium chelate derivatives, Reactive Red 4, BODIPY dyes and cyanine dyes.

The compound may also be connected to an affinity tag, as discussed above. The affinity tag is capable of attachment to a target protein. By adding such a conjugate to a biological mixture, it is possible to detect the presence or absence of an analyte such as a target protein, by monitoring a luminescence emission of the conjugate mixture in response to an excitation energy. One of skill in the art will realize that the order of addition of the various assay components can be varied. In other words, the assay components can be admixed simultaneously, stepwise, sequentially in any order, or any combination thereof.

In certain other aspects, the methods and compounds of the present invention can be used with a number of other analytical techniques to determine protein identity. These include for example, iterative labeling reagents specific for a particular amino acid, 2-D electrophoresis (2DE), single or multidimensional HPLC and capillary electrophoresis. Although the present disclosure exemplifies ICAT reagents this in no way should be construed as limiting. For example, in certain aspects, the present compositions and methods label all peptides in a sample having lysines residues for further analysis. Thus, the labels of the present invention are far superior to prior art techniques as there is a far higher percentage of lysine residues than cysteine residues. Further, non-lysine containing peptides can be modified with a second label at their N-terminus. This process enables the two residues to be distinguished.

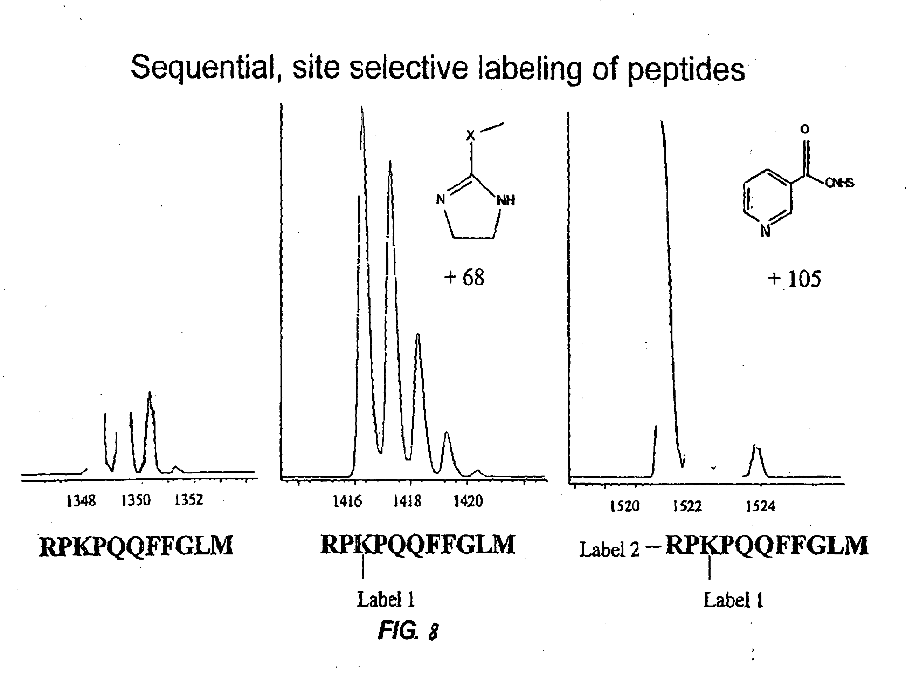

The chemical specificity of the labels of the present invention for lysine amines rather than N-termini allow for other labeling techniques to be done where one type of label is first put on lysines residues, and then an entirely different label is placed on the N-termini. For example, with reference to figure 8, the first panel shows an unmodified peptide. The middle panel shows the peptide with a lysine label of the present invention having a 28 Da difference. Finally, the n-terminus is labeled with a specific n-termini label. As such, different labels can be put on each end of a given peptide (each of which has their desired property and/or isotopically unique pattern). This allows for unequivocal determination if the N-terminus is blocked, identification of cleavage sites, fragmenting peptides in different manners as desired and the like.

For example, the determination of whether the *N*-terminus of a protein was blocked (*i*.*e*., as a result of the common post-translational modification of acylation) is performed in the following manner: All lysine residues are reacted with the compound of the present invention in such a manner that the total number of lysines is determined in order to assist in protein identification. The unreacted *N*-termini of all the peptides are then subsequently labeled with isotopic versions of a label having a mass difference that is an odd (vs. an even) number (for example, the H3 and D3 forms of the *N*-hydroxysuccinimide esters of acetic acid). Any peptide with a free *N*-terminal amino group exists as a pair of isotopically labeled peptides with an odd mass difference, while any *N*-terminal peptide with a blocked amino group exists as a pair of isotopically labeled peptides with an even mass difference, or as a single species (in the case of a peptide having no lysine residues). Focusing data analysis on species having an even or no mass difference between their isotopically labeled constituents enables the identification of proteins with *N*-blocked termini.

In an alternative embodiment, all lysine residues in a polypeptide are reacted with a compound of the present invention in such a manner that the total number of lysines in the polypeptide sample are blocked. The free amine on the N-terminus can then be used to tether the molecule, for example on a solid support, such as an affinity column.

### F. EXAMPLES

### MATERIALS AND METHODS

### Materials

Equine myoglobin, model and calibration standard polypeptides, and sequencing grade trypsin were purchased from Sigma (St. Louis, MO, USA). All other chemicals were purchased from Aldrich (Milwaukee, WI, USA). Solvents for MALDI MS sample preparation were HPLC grade unless otherwise noted. All chemicals were used as received.

### Synthesis of labels

In addition to their labeling behavior, all labels were also chemically characterized. Nuclear magnetic resonance (NMR) spectra were acquired using a Bruker DRX-400. Mass spectra were obtained using a Hewlett-Packard LC/MSD with direct infusion of the sample.

4,5-tetradeutero-imidazolidine-2-thione (**1b**): (Allen CFH *et al., J. Organic Synthethes* 3:394) To a 10 mL round bottom flask equipped with a magnetic stir bar and a reflux condenser was added 1,2-tetradeuteroethylenediamine (1.00 g, 15.6 mmol), absolute ethanol (3.0 mL), and deionized water (3.0 mL). Carbon disulfide (0.94 mL, 15.6 mmol) was slowly added drop wise to the stirring solution at room temperature. The stirred solution was refluxed for 2 hours, and then cooled to room temperature. Concentrated HCl (0.15 mL) was added, and the reaction mixture was refluxed for 16 hours. As the solution cooled to room temperature, white crystals formed, and the reaction mixture was then kept at 4 °C overnight. The white solid was filtered, washed with cold (-20 °C) acetone, and dried to obtain 1.14 g of the title compound as white crystals (69%). ¹H-NMR (400 MHz, DMSO-d₆) δ [ppm] 7.96 (s, 2H). MS (Electrospray) 106.1 (M+)

2-methylthioyl-4,5-tetradeutero-1H-imidazole hydroiodide (**2b**): A 20 mL threaded amber glass vial fitted with a magnetic stir bar was charged with 4,5-tetradeutero-imidazolidine-2-thione (**1b**) (920 mg, 8.68 mmol) and the minimum volume of methanol required to dissolve the starting material (∼5 mL). The vial was sealed with a rubber septum, and iodomethane (0.56 mL, 9.00 mmol) was added through a syringe. After the reaction mixture was stirred at room temperature for 48 h, the solvent was removed *in-vacuo.* The crude product was triturated in ether, the solids were filtered in the absence of light, washed with ether, and dried to afford 1.54 g of the desired product as a light yellow powder (85%). ¹H-NMR (400 MHz, DMSO-d₆) δ [ppm] 2.64 (s, 3H). MS (Electrospray) 117.2 (M+).

2-methoxy-4,5-dihydro-1*H*-imidazole **(3a)** (Cain CK et al., J. Org. Chem. 22:1283 (1957)). To a 100 mL round bottom flask equipped with a magnetic stir bar and condenser was added 2-methylthio-2-imadazoline hydroiodide (commercially available, 5.0 g, 20.4 mmol), 25 wt.% sodium methoxide in methanol (14 mL, 57.0 mmol NaOCH₃) and methanol (20 mL). The stirred solution was refluxed overnight. The solvent was removed *in-vacuo*, and the solid was redissolved in saturated brine. The aqueous solution was extracted five times with dichloromethane. The organic extracts were combined, dried over magnesium sulfate, filtered, and dried *in-vacuo*. The crude product was triturated in ether, the solids were filtered, washed with ether, and dried to afford 1.42 g of the desired product as a white powder (70%). ¹H-NMR (400 MHz, DMSO-d₆) δ [ppm] 3.82 (s, 3H), 3.59 (s, 4H) MS (Electrospray) 101.1 (M+).

The deuterated analog 2-methoxy-4,5-tetradeutero-1H-imidazole (**3b**) was made in an identical matter starting from (2b). MS (Electrospray) 105.1 (M+).

### Derivatization of polypeptides

20 µL of polypeptide solution (∼100 µM) in 150 µL of distilled water was incubated overnight at 50°C with 30 µL of 1.5M 2-methoxy-4,5-dihydro-1*H*-imidazole. The pH of the reaction solution was basic as measured with colorpHast strips (EM Sciences, Gibbstown, NJ, USA). Polypeptide solutions were also labeled with *O*-methylisourea following the method of Reilly *et al*. (Beardsley RL et al., Rapid Commun. Mass Spectrom. 14:2147 (2000)). The samples were acidified with glacial acetic acid, and cleaned using C₁₈ Zip Tips^{™} purchased from Millipore (Bedford, MA, USA). The labeled polypeptides were eluted with 10 µL of 50:50 acetonitrile:water (v:v)with 0.1% trifluoroacetic acid for ESI tandem MS studies or 2 µL of a 50 mg/ml solution of 2,5-dihydroxybenzoic acid in the same solvent system directly onto a target plate for MALDI MS.

### Mass spectrometry and data processing

Tandem mass spectra were acquired using a Finnigan (Thermoquest, San Jose, CA) LCQ-DECA mass spectrometer equipped with a home built µESI source. Samples were introduced by direct infusion at a flow rate of 1 µL/minute. Collisonal activation was performed manually using a 4-Da isolation window and an activation amplitude that ensures at least a 90% reduction in the signal of the parent ion (typical values were 32-34%).

The MALDI mass spectra of the tryptic digests of myoglobin were acquired on a 7 T Bruker Apex™ II FT-ICR (Fourier transform ion cyclotron resonance) equipped with a intermediate pressure MALDI source equipped with a N₂ laser. Standard polypeptides (bradykinin, angiotensin I, substance P, neurotensin, ACTH 18-39, melittin, and insulin B chain) were used as internal calibrants. The recalibration and data reduction were performed automatically using THRASH (Hom DM et al., J. Am. Soc. Mass Spectrom. 11:320 (2000)). The resulting masses were assigned to polypeptide sequences from myoglobin using PAWS (Protcometrics, New York. NY).

### COMPARATIVE EXAMPLE

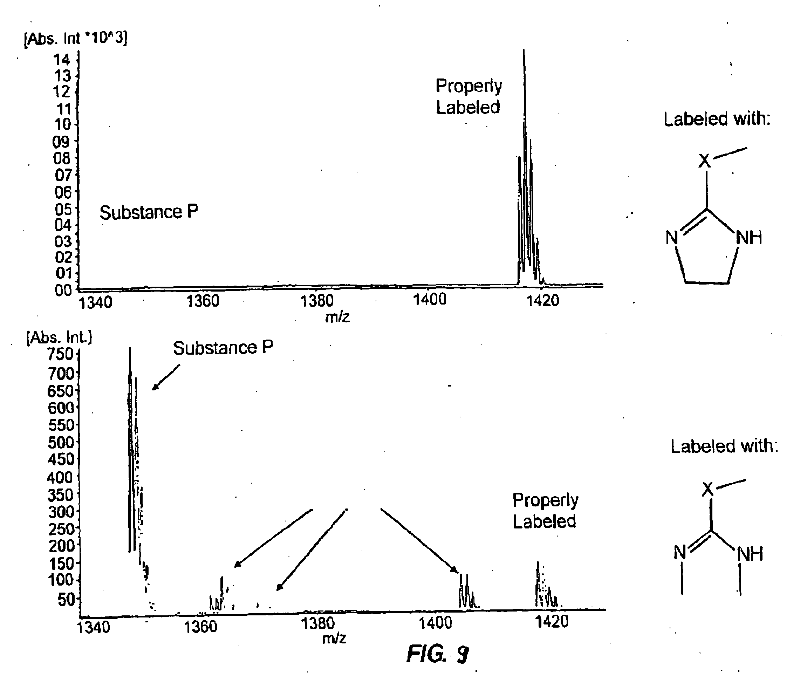

Figure 9 shows a comparative example of differential quantitation using a compound of the present invention versus a comparative label *N*-*N*'-dimethyl-*O-*methylisourea. One method to perform differential quantitation measurements while maintaining the ionization benefits afforded by *N*-*N*'-dimethyl-*O*-methylisourea without resorting to the relatively expensive ¹³C and ¹⁵N stable isotope version of this species, involve using a label that maintains a similar reactivity profile, but that also possesses sites for the stable incorporation of either hydrogen or deuterium. However, this cannot be achieved through simple alkyl substitution of *N*-*N*'-dimethyl-*O*-methylisourea.

Figure 9 shows the spectra of a peptide reacted with inventive 2-methoxy-4,5-dihydro-1H-imidazole and comparative *N*-*N*'-dimethyl-*O*-methylisourea. The comparative molecule was prepared from its commercially available 1,3-dimethyl-2-thiourea analog and the labeling reactions were performed under identical conditions.

The results indicate that the peptide with the label of this invention afforded the expected product. The reaction yield with the comparative label was lower (to a much lesser extent), and more detrimentally, resulted in the production of a number of other species in the spectra compared to the inventive compound. These additional unwanted peaks serve only to increase the complexity of the spectra and the difficulty of its interpretation.

## Claims

1. A method for mass spectrometric polypeptide analysis, said method comprising:
a) ionizing a modified polypeptide having a lysine residue of Formula Ib 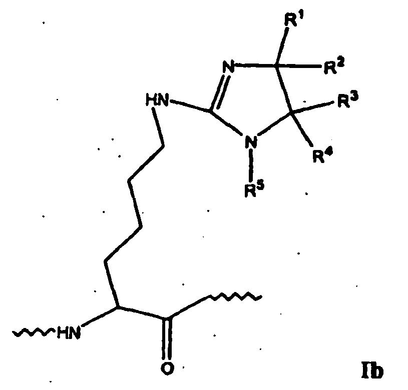 wherein R¹, R², R³ and R⁴ are each independently a member selected from the group consisting of hydrogen, deuterium, halogen, hydroxyl, alkyl, alkoxy, aryl, and an affinity tag; or, alternatively, R², R³ and the carbons to which they are attached join to form an *n*-membered carbocyclic, heterocyclic, aryl or heteroaryl ring;
*n* is an integer from 4 to 8;
R⁵ is a member selected from the group consisting of hydrogen, halogen, hydroxyl, alkyl, alkoxy, and aryl, to form an ionized polypeptide; and
b) analyzing the results of the ionized modified polypeptide.

2. The method for mass spectrometric polypeptide analysis according to claim 1, wherein R¹, R², R³ and R⁴ are each hydrogen; wherein R¹, R², R³ and R⁴ are each deuterium; wherein R¹ and R² are each alkyl.

3. The method for mass spectrometric polypeptide analysis according to claim 1, or 2, further comprising enzymatically digesting said modified polypeptide prior to step (a).

4. The method for mass spectrometric polypeptide analysis according to claim 1, 2 or 3,
wherein R¹ is an affinity tag, and said method further comprises purifying said modified polypeptide using said affinity tag prior to step (a).

5. The method for mass spectrometric polypeptide analysis according to claim 1, 2, 3 or 4, wherein the ionization of step (a) comprises matrix-assisted desorption/ionization mass spectrometry; or wherein the ionization of step (a) comprises electrospray ionization mass spectrometry.

6. A method for analyzing a sequentially labeled polypeptide, said method comprising:
incubating said polypeptide with a first label to form a first labeled polypeptide;
incubating said first labeled polypeptide with a second label to form a second labeled polypeptide, wherein at least one of said first label or said second label is a compound having Formula IIa 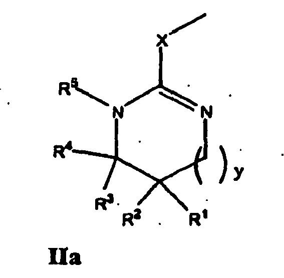
wherein R¹, R², R³ and R⁴ are each independently a member selected from the group consisting of hydrogen, deuterium, halogen, hydroxyl, alkyl, alkoxy, aryl, and an affinity tag; or, alternatively, R², R³ and the carbons to which they are attached join to form an *n*-membered carbocyclic, heterocyclic, aryl or heteroaryl ring;
*n* is an integer from 4 to 8;
R⁵ is a member selected from the group consisting of hydrogen, halogen, hydroxyl, alkyl, alkoxy, optionally substituted aryl and an affinity tag;
X is a heteroatom selected from the groups consisting of O and S;
y is 0; and
analyzing said second labeled polypeptide with a mass spectrometer.

7. The method for analyzing a sequentially labeled polypeptide according to claim 6, further comprising labeling said second labeled polypeptide with a third label to form a third labeled polypeptide.

8. The method for analyzing a sequentially labeled polypeptide according to claim 7, wherein said first label, said second label and said third label are each independently selected from the group consisting of a compound of Formula IIa, and an amino acid specific label.

9. The method for analyzing a sequentially labeled polypeptide according to claim 8, wherein said amino acid specific label is a member selected from the group consisting of a cysteine labeling reagent, an arginine label reagent and a carboxylic acid labeling reagent.

10. The method for analyzing a sequentially labeled polypeptide according to claim 9, wherein said carboxylic acid labelling reagent is an alcohol under esterifying conditions to form an ester.

11. The method for analyzing a sequentially labeled polypeptide according to claim 6, 7, 8, 9 or 10, wherein said high mass accuracy mass spectrometer is a Fourier transform ion cyclotron resonance spectrometer.

12. method for increasing the ionization efficiency of a lysine-containing polypeptide, said method comprising:
a) incubating a lysine-containing polypeptide with a compound of Formula IIa 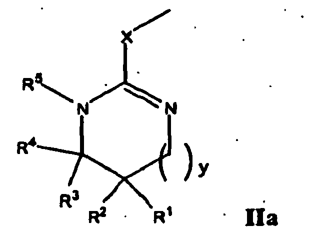 to form a modified polypeptide having a lysine residue of Formula Ib: 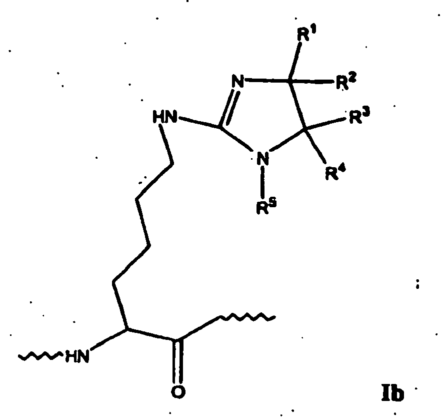 R¹, R², R³ and R⁴ are each independently a member selected from the group consisting of hydrogen, deuterium, halogen, hydroxyl, alkyl, alkoxy, aryl, and an affinity tag; or, alternatively, R², R³ and the carbons to which they are attached join to form an n-membered carbocyclic, heterocyclic, aryl or heteroaryl ring;
*n* is an integer from 4 to 8;
R⁵ is a member selected from the group consisting of hydrogen, halogen, hydroxyl, alkyl, alkoxy, and aryl, to form an ionized polypeptide;
X is a heteroatom selected from the groups consisting of O and S;
y is 0; and
b) ionizing said modified polypeptide having a lysine residue of Formula Ib, thereby increasing the ionization efficiency of said lysine-containing polypeptide.

13. A method for performing differential quantitation of a lysine-containing polypeptide, said method comprising:
a) incubating a first sample of a lysine-containing polypeptide with a first isotopic version of a compound of Formula IIa 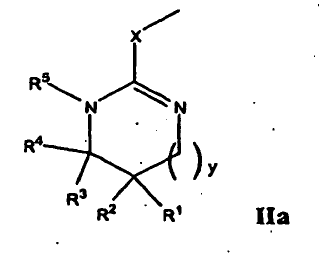 to form a first modified polypeptide having a lysine residue of Formula Ib: 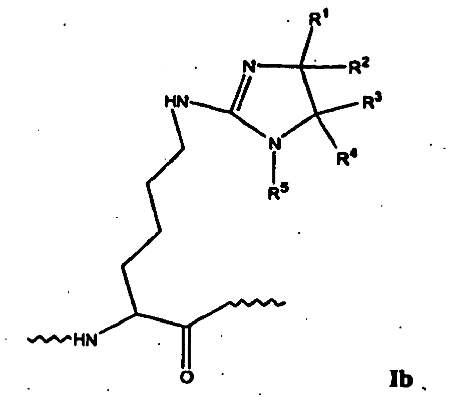 R¹, R², R³ and R⁴ are each independently a member selected from the group consisting of hydrogen, deuterium, halogen, hydroxyl, alkyl, alkoxy, aryl, and an affinity tag; or, alternatively, R², R³ and the carbons to which they are attached join to form an n-membered carbocyclic, heterocyclic, aryl or heteroaryl ring;
*n* is an integer from 4 to 8;
R⁵ is a member selected from the group consisting of hydrogen, halogen, hydroxyl, alkyl, alkoxy, and aryl, to form an ionized polypeptide;
X is a heteroatom selected from the groups consisting of O and S;
y is 0; and
b) incubating a second sample of said lysine-containing polypeptide with a second isotopic version of a compound of Formula IIa to form a second modified polypeptide having a lysine residue of Formula Ib;
c) combining said first sample and said second sample of said modified polypeptide having a lysine residue of Formula Ib to form a mixture; and
d) ionizing said mixture to form a series of isotopically labeled polypeptide pairs, wherein the relative concentrations of said first modified polypeptide having a lysine residue of Formula Ib and said second modified polypeptide having a lysine residue of Formula Ib are directly proportional to their relative signal intensities, and wherein the mass difference between the two modified polypeptides indicates the number of lysine(s) that are present in said lysine-containing polypeptide.

14. The method for performing differential quantitation of a lysine-containing polypeptide according to claim 13, wherein said series of isotopically labeled polypeptide pairs indicates that at least two lysines are present.

15. The method for performing differential quantitation of a lysine-containing polypeptide according to claim 13 or 14, wherein said first sample and said second sample of said lysine-containing polypeptides are combined in equal proportions.

16. A method for determining the number of lysines in a lysine-containing polypeptide, said method comprising:
a) incubating a sample of a lysine-containing polypeptide with a first isotopic version of a compound of Formula IIa and a second isotopic version of a compound of Formula IIa to form a mixture having a first modified polypeptide having a lysine residue of Formula Ib and a second modified polypeptide having a lysine residue of Formula Ib, wherein Formula Ib has the formula: 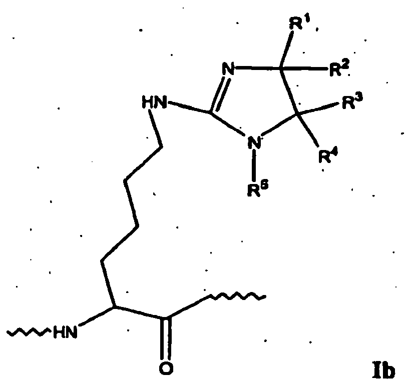 R¹, R², R³ and R⁴ are each independently a member selected from the group consisting of hydrogen, deuterium, halogen, hydroxyl, alkyl, alkoxy, aryl, and an affinity tag; or, alternatively, R², R³ and the carbons to which they are attached join to form an n-membered carbocyclic, heterocyclic, aryl or heteroaryl ring;
*n* is an integer from 4 to 8;
R⁵ is a member selected from the group consisting of hydrogen, halogen, hydroxyl, alkyl, alkoxy, and aryl, to form an ionized polypeptide; and
b) ionizing said mixture to form a series of isotopically labeled polypeptide pairs, wherein the mass difference between said first modified polypeptide having a lysine residue of Formula Ib and said second modified polypeptide having a lysine residue of Formula Ib indicates the number of lysine(s) that are present in said lysine-containing polypeptide.

17. The method for determining the number of lysines in a lysine-containing polypeptide according to 16, wherein said series of isotopically labeled polypeptide pairs indicates that at least two lysines are present.

## Patentansprüche

1. Verfahren zur massenspektrometrischen Analyse von Polypeptiden, wobei das Verfahren umfasst:
a) Ionisieren eines modifizierten Polypeptids, das einen Lysinrest der Formel Ib enthält 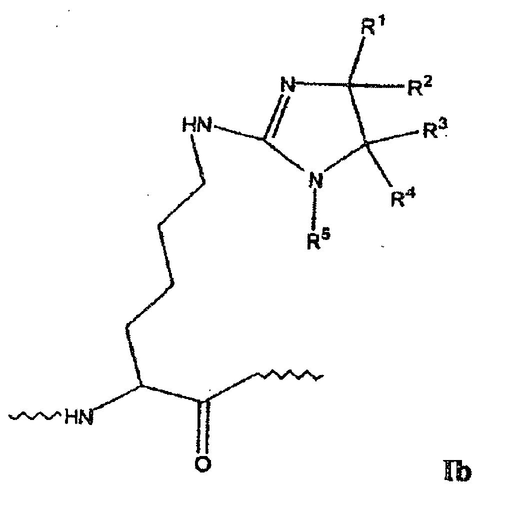 wobei R¹, R², R³ und R⁴ jeweils ein Element sind, das aus der Gruppe bestehend aus Wasserstoff, Deuterium, Halogen, Hydroxyl, Alkyl, Alkoxy, Aryl und einem Affinitäts-Tag gewählt ist; oder, alternativ, R², R³ und die Kohlenstoffe, an die sie gebunden sind, sich zu einem n-gliedrigen carbozyklischen, heterozyklischen Aryl- oder Heteroaryl-Ring verbinden;
n eine Ganzzahl im Bereich 4 bis 8 ist;
R⁵ ein Element ist, das aus der Gruppe bestehend aus Wasserstoff, Halogen, Hydroxyl, Alkyl, Alkoxy und Aryl gewählt ist, um ein ionisiertes Polypeptid zu bilden; und
b) Analysieren der Ergebnisse des ionisierten, modifizierten Polypeptids.

2. Verfahren zur massenspektrometrischen Analyse von Polypeptiden nach Anspruch 1, wobei R¹, R², R³ und R⁴ jeweils Wasserstoff sind; wobei R¹, R², R³ und R⁴ jeweils Deuterium sind; wobei R¹ und R² jeweils Alkyl sind.

3. Verfahren zur massenspektrometrischen Analyse von Polypeptiden nach Anspruch 1 oder 2, ferner umfassend das enzymatische Verdauen des modifizierten Polypeptids vor Schritt (a).

4. Verfahren zur massenspektrometrischen Analyse von Polypeptiden nach Anspruch 1, 2 oder 3, wobei R¹ ein Affinitäts-Tag ist und das Verfahren ferner umfasst, das modifizierte Polypeptid mithilfe des Affinitäts-Tags vor Schritt (a) aufzureinigen.

5. Verfahren zur massenspektrometrischen Analyse von Polypeptiden nach Anspruch 1, 2, 3 oder 4, wobei die Ionisierung in Schritt (a) die matrixgestützte Desorptions- /Ionisations-Massenspektrometrie umfasst oder wobei die Ionisierung von Schritt (a) die Elektrospray-Ionisations-Massenspektrometrie umfasst.

6. Verfahren zum Analysieren eines sequenziell markierten Polypeptids, wobei das Verfahren umfasst:
Inkubieren des Polypeptids mit einem ersten Label, um ein erstes markiertes Polypeptid zu bilden;
Inkubieren des ersten markierten Polypeptids mit einem zweiten Label, um ein zweites markiertes Polypeptid zu bilden, wobei mindestens entweder das erste oder das zweite Label eine Zusammensetzung ist, die die Formel IIa aufweist 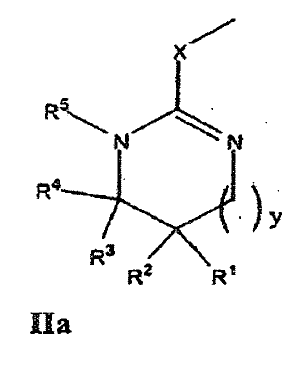
wobei R¹, R², R³ und R⁴ jeweils ein Element sind, das aus der Gruppe bestehend aus Wasserstoff, Deuterium, Halogen, Hydroxyl, Alkyl, Alkoxy, Aryl und einem Affinitäts-Tag gewählt ist; oder, alternativ, R², R³ und die Kohlenstoffe, an die sie gebunden sind, sich zu einem n-gliedrigen carbozyklischen, heterozyklischen Aryl- oder Heteroaryl-Ring verbinden;
n eine Ganzzahl im Bereich 4 bis 8 ist;
R⁵ ein Element ist, das aus der Gruppe, die aus Wasserstoff, Halogen, Hydroxyl, Alkyl, Alkoxy, wahlweise substituiertem Aryl und einem Affinitäts-Tag besteht, gewählt ist;
X ein Heteroatom ist, das aus den Gruppen, die aus O und S bestehen, gewählt ist;
y gleich 0 ist; und
Analysieren des zweiten markierten Polypeptids mit einem Massenspektrometer.

7. Verfahren zum Analysieren eines sequenziell markierten Polypeptids nach Anspruch 6, ferner umfassend das Markieren des zweiten markierten Polypeptids mit einem dritten Label, um ein drittes markiertes Polypeptid zu bilden.

8. Verfahren zum Analysieren eines sequenziell markierten Polypeptids nach Anspruch 7, wobei das erste Label, das zweite Label und das dritte Label jeweils unabhängig aus der Gruppe bestehend aus einer Zusammensetzung der Formel IIa und einem aminosäurespezifischen Label gewählt sind.

9. Verfahren zum Analysieren eines sequenziell markierten Polypeptids nach Anspruch 8, wobei das aminosäurespezifische Label ein Element ist, das aus der Gruppe bestehend aus einem Cystein-Markierungs-Reagens, einem Arginin-Markierungs-Reagens und einem Carboxylsäure-Markierungs-Reagens gewählt ist.

10. Verfahren zum Analysieren eines sequenziell markierten Polypeptids nach Anspruch 9, wobei das Carboxylsäure-Markierungs-Reagens ein Alkohol unter Esterifizierungsbedingungen ist, um einen Ester zu bilden.

11. Verfahren zum Analysieren eines sequenziell markierten Polypeptids nach Anspruch 6, 7, 8, 9 oder 10, wobei das hoch massengenaue Massenspektrometer ein Fourier-Transform-Ionenzyklotronenresonanz-Spektrometer ist.

12. Verfahren zum Erhöhen der Ionisierungseffizienz eines lysinhaltigen Polypeptids, wobei das Verfahren umfasst:
a) Inkubieren eines lysinhaltigen Polypeptids mit einer Zusammensetzung der Formel IIa 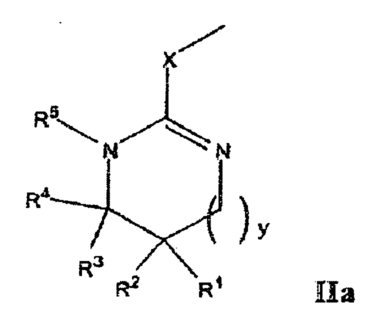 um ein modifiziertes Polypeptid zu bilden, das einen Lysinrest der Formel Ib enthält: 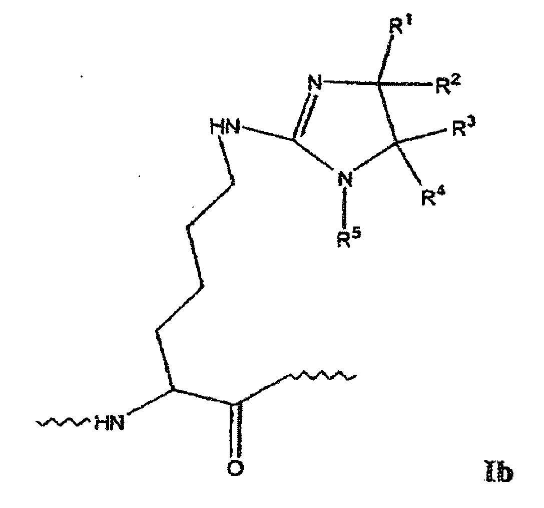 R¹, R², R³ und R⁴ ist jeweils ein Element, das aus der Gruppe, die aus Wasserstoff, Deuterium, Halogen, Hydroxyl, Alkyl, Alkoxy, Aryl und einem Affinitäts-Tag besteht, gewählt ist; oder, alternativ, verbinden sich R², R³ und die Kohlenstoffe, an die sie gebunden sind, zu einem n-gliedrigen carbozyklischen, heterozyklischen Aryl- oder Heteroaryl-Ring;
n ist eine Ganzzahl im Bereich 4 bis 8;
R⁵ ist ein Element, das aus der Gruppe, die aus Wasserstoff, Halogen, Hydroxyl, Alkyl, Alkoxy und Aryl besteht, gewählt ist, um ein ionisiertes Polypeptid zu bilden;
X ist ein Heteroatom, das aus den Gruppen, die aus O und S bestehen, gewählt ist; y ist gleich 0; und
b) Ionisieren des modifizierten Polypeptids mit einem Lysinrest der Formel Ib, wodurch die Ionisationseffizienz des lysinhaltigen Polypeptids erhöht wird.

13. Verfahren zum Durchführen einer differenziellen Quantifizierung eines lysinhaltigen Polypeptids, wobei das Verfahren umfasst:
a) Inkubieren einer ersten Probe eines lysinhaltigen Polypeptids mit einer ersten isotopischen Version einer Zusammensetzung der Formel IIa 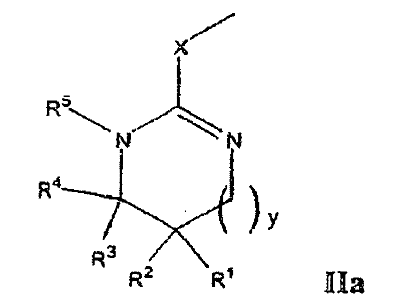 um ein erstes modifiziertes Polypeptid zu bilden, das einen Lysinrest der Formel Ib enthält: 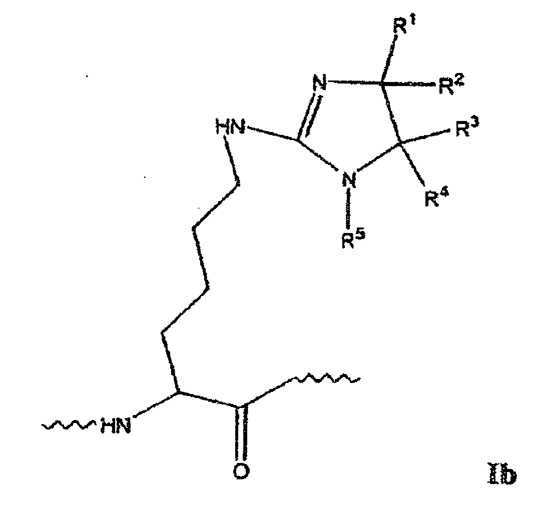 R¹, R², R³ und R⁴ ist jeweils ein Element, das aus der Gruppe, die aus Wasserstoff, Deuterium, Halogen, Hydroxyl, Alkyl, Alkoxy, Aryl und einem Affinitäts-Tag besteht, gewählt ist; oder, alternativ, verbinden sich R², R³ und die Kohlenstoffe, an die sie gebunden sind, zu einem n-gliedrigen carbozyklischen, heterozyklischen Aryl- oder Heteroaryl-Ring;
n ist eine Ganzzahl im Bereich 4 bis 8;
R⁵ ist ein Element, das aus der Gruppe, die aus Wasserstoff, Halogen, Hydroxyl, Alkyl, Alkoxy und Aryl besteht, gewählt ist;
X ist ein Heteroatom, das aus den Gruppen, die aus O und S bestehen, gewählt ist; y ist gleich 0; und
b) Inkubieren einer zweiten Probe eines lysinhaltigen Polypeptids mit einer zweiten isotopischen Version einer Zusammensetzung der Formel IIa, um ein zweites modifiziertes Polypeptid zu bilden, das einen Lysinrest der Formel Ib enthält;
c) Kombinieren der ersten Probe und der zweiten Probe des modifizierten Polypeptids, das einen Lysinrest der Formel Ib aufweist, um eine Mischung zu bilden; und
d) Ionisieren der Mischung, um eine Reihe von isotopisch markierten Polypeptid-Paaren zu bilden, wobei die relativen Konzentrationen des ersten modifizierten Polypeptids, das einen Lysinrest der Formel Ib aufweist, und des zweiten modifizierten Polypeptids, das einen Lysinrest der Formel Ib aufweist, sich direkt proportional zu ihren jeweiligen Signalintensitäten verhalten, und wobei der Massenunterschied zwischen den beiden modifizierten Polypeptiden die Anzahl von Lysinen anzeigt, die in dem lysinhaltigen Polypeptid enthalten sind.

14. Verfahren zum Durchführen einer differenziellen Quantifizierung eines lysinhaltigen Polypeptids nach Anspruch 13, wobei die Reihe von isotopisch markierten Polypeptid-Paaren anzeigt, dass zumindest zwei Lysine vorhanden sind.

15. Verfahren zum Durchführen einer differenziellen Quantifizierung eines lysinhaltigen Polypeptids nach Anspruch 13 oder 14, wobei die erste Probe und die zweite Probe der lysinhaltigen Polypeptide in gleichen Anteilen kombiniert sind.

16. Verfahren zum Bestimmen der Anzahl von Lysinen in einem lysinhaltigen Polypeptid, wobei das Verfahren umfasst:
a) Inkubieren einer Probe eines lysinhaltigen Polypeptids mit einer ersten isotopischen Version einer Zusammensetzung der Formel IIa und einer zweiten isotopischen Version einer Zusammensetzung der Formel IIa, um eine Mischung zu bilden, die ein erstes modifiziertes Polypeptid, das einen Lysinrest der Formel Ib aufweist, und ein zweites modifiziertes Polypeptid, das einen Lysinrest der Formel Ib aufweist, enthält, wobei die Formel Ib folgende Formel hat: 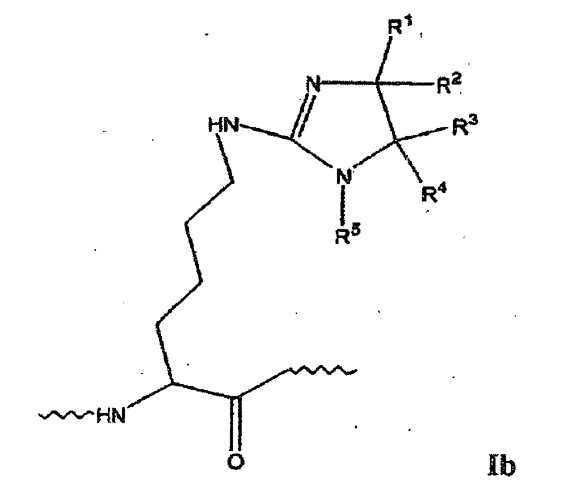 R¹, R², R³ und R⁴ ist jeweils ein Element, das aus der Gruppe, die aus Wasserstoff, Deuterium, Halogen, Hydroxyl, Alkyl, Alkoxy, Aryl und einem Affinitäts-Tag besteht, gewählt ist; oder, alternativ, verbinden sich R², R³ und die Kohlenstoffe, an die sie gebunden sind, zu einem n-gliedrigen carbozyklischen, heterozyklischen Aryl- oder Heteroaryl-Ring;
n ist eine Ganzzahl im Bereich 4 bis 8;
R⁵ ist ein Element, das aus der Gruppe, die aus Wasserstoff, Halogen, Hydroxyl, Alkyl, Alkoxy und Aryl besteht, gewählt ist, um ein ionisiertes Polypeptid zu bilden; und
b) Ionisieren der Mischung, um eine Reihe von isotopisch markierten Polypeptid-Paaren zu bilden, wobei der Massenunterschied zwischen dem ersten modifizierten Polypeptid, das einen Lysinrest der Formel Ib aufweist, und dem zweiten modifizierten Polypeptid, das einen Lysinrest der Formel Ib aufweist, die Anzahl von Lysinen anzeigt, die in dem lysinhaltigen Polypeptid enthalten sind.

17. Verfahren zum Bestimmen der Anzahl von Lysinen in einem lysinhaltigen Polypeptid nach Anspruch 16, wobei die Reihe von isotopisch markierten Polypeptid-Paaren anzeigt, dass zumindest zwei Lysine vorhanden sind.

## Revendications

1. Un procédé d'analyse de polypeptides par spectrométrie de masse, ledit procédé comprenant :
a) l'ionisation d'un polypeptide modifié possédant un résidu lysine de la Formule Ib 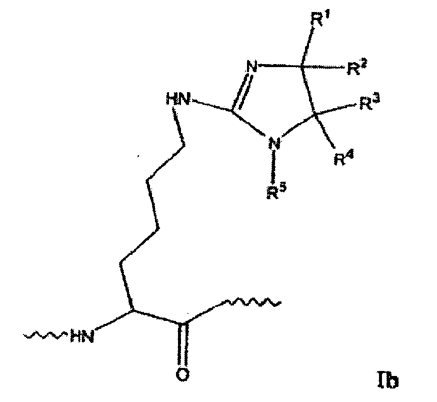 où R¹, R², R³ et R⁴ sont chacun indépendamment un membre sélectionné dans le groupe se composant de hydrogène, deutérium, halogène, hydroxyle, alkyle, alkoxy, aryle et d'un marqueur d'affinité, ou, dans une variante, R², R³ et les carbones auxquels ils sont attachés se joignent pour former un noyau carbocyclique, hétérocyclique, aryle ou hétéroaryle à *n* chaînons,
*n* est un entier de 4 à 8,
R⁵ est un membre sélectionné dans le groupe se composant de hydrogène, halogène, hydroxyle, alkyle, alkoxy, et aryle de façon à former un polypeptide ionisé, et
b) l'analyse des résultats du polypeptide modifié ionisé.

2. Le procédé d'analyse de polypeptides par spectrométrie de masse selon la revendication 1, où R¹, R², R³ et R⁴ sont chacun hydrogène, où R¹, R², R³ et R⁴ sont chacun deutérium, où R¹ et R² sont chacun alkyle.

3. Le procédé d'analyse de polypeptides par spectrométrie de masse selon la revendication 1 ou 2, comprenant en outre la digestion enzymatique dudit polypeptide modifié avant l'opération (a).

4. Le procédé d'analyse de polypeptides par spectrométrie de masse selon la revendication 1, 2 ou 3, où R¹ est un marqueur d'affinité, et ledit procédé comprend en outre la purification dudit polypeptide modifié au moyen dudit marqueur d'affinité avant l'opération (a).

5. Le procédé d'analyse de polypeptides par spectrométrie de masse selon la revendication 1, 2, 3 ou 4, où l'ionisation de l'opération (a) comprend une spectrométrie de masse à désorption/ionisation assistée par matrice ou où l'ionisation de l'opération (a) comprend une spectrométrie de masse à ionisation par électropulvérisation.

6. Un procédé d'analyse d'un polypeptide marqué séquentiellement, ledit procédé comprenant :
l'incubation dudit polypeptide avec un premier marqueur de façon à former un premier polypeptide marqué,
l'incubation dudit premier polypeptide marqué avec un deuxième marqueur de façon à former un deuxième polypeptide marqué, où au moins un dudit premier marqueur ou dudit deuxième marqueur est un composé possédant la Formule IIa 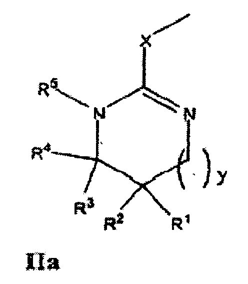
où R¹, R², R³ et R⁴ sont chacun indépendamment un membre sélectionné dans le groupe se composant de hydrogène, deutérium, halogène, hydroxyle, alkyle, alkoxy, aryle et d'un marqueur d'affinité, ou, dans une variante, R², R³ et les carbones auxquels ils sont attachés se joignent pour former un noyau carbocyclique, hétérocyclique, aryle ou hétéroaryle à n chaînons,
*n* est un entier de 4 à 8,
R⁵ est un membre sélectionné dans le groupe se composant de hydrogène, halogène, hydroxyle, alkyle, alkoxy, et aryle facultativement substitué et d'un marqueur d'affinité,
X est un hétéroatome sélectionné dans les groupes se composant de O et S,
y est 0, et
l'analyse dudit deuxième polypeptide marqué avec un spectromètre de masse.

7. Le procédé d'analyse d'un polypeptide marqué séquentiellement selon la revendication 6, comprenant en outre le marquage dudit deuxième polypeptide marqué avec un troisième marqueur de façon à former un troisième polypeptide marqué.

8. Le procédé d'analyse d'un polypeptide marqué séquentiellement selon la revendication 7, où ledit premier marqueur, ledit deuxième marqueur et ledit troisième marqueur sont chacun indépendamment sélectionnés dans le groupe se composant d'un composé de la Formule IIa, et d'un marqueur spécifique aux acides aminés.

9. Le procédé d'analyse d'un polypeptide marqué séquentiellement selon la revendication 8, où ledit marqueur spécifique aux acides aminés est un membre sélectionné dans le groupe se composant d'un réactif de marquage de la cystéine, d'un réactif de marquage de l'arginine et d'un réactif de marquage de l'acide carboxylique.

10. Le procédé d'analyse d'un polypeptide marqué séquentiellement selon la revendication 9, où ledit réactif de marquage de l'acide carboxylique est un alcool dans des conditions d'estérification de façon à former un ester.

11. Le procédé d'analyse d'un polypeptide marqué séquentiellement selon la revendication 6, 7, 8, 9 ou 10, où ledit spectromètre de masse à précision de masse élevée est un spectromètre par résonance cyclotronique ionique à transformée de Fourier.

12. Un procédé d'accroissement de l'efficacité d'ionisation d'un polypeptide contenant de la lysine, ledit procédé comprenant :
a) l'incubation d'un polypeptide contenant de la lysine avec un composé de la Formule IIa 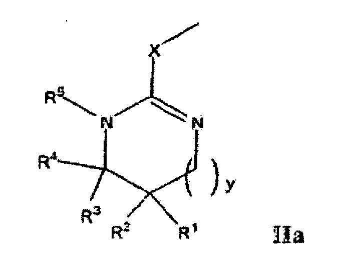 de façon à former un polypeptide modifié possédant un résidu lysine de la Formule Ib : 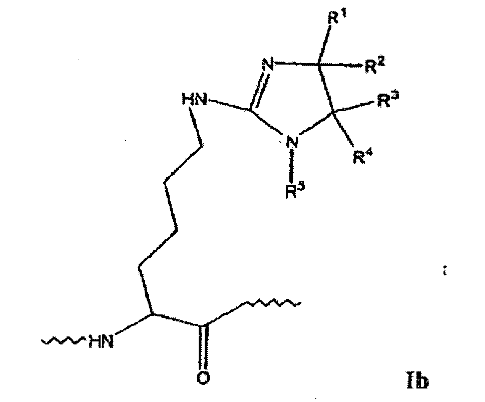 R¹, R², R³ et R⁴ sont chacun indépendamment un membre sélectionné dans le groupe se composant de hydrogène, deutérium, halogène, hydroxyle, alkyle, alkoxy, aryle et d'un marqueur d'affinité, ou, dans une variante, R², R³ et les carbones auxquels ils sont attachés se joignent pour former un noyau carbocyclique, hétérocyclique, aryle ou hétéroaryle à *n* chaînons,
*n* est un entier de 4 à 8,
R⁵ est un membre sélectionné dans le groupe se composant de hydrogène, halogène, hydroxyle, alkyle, alkoxy, et aryle de façon à former un polypeptide ionisé,
X est un hétéroatome sélectionné dans les groupes se composant de O et S,
y est 0, et
b) l'ionisation dudit polypeptide modifié possédant un résidu lysine de la Formule Ib, accroissant ainsi l'efficacité d'ionisation dudit polypeptide contenant de la lysine.

13. Un procédé d'exécution d'une quantification différentielle d'un polypeptide contenant de la lysine, ledit procédé comprenant :
a) l'incubation d'un premier échantillon d'un polypeptide contenant de la lysine avec une première version isotopique d'un composé de la Formule IIa 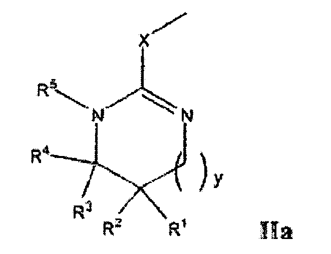 de façon à former un premier polypeptide modifié possédant un résidu lysine de la Formule Ib : 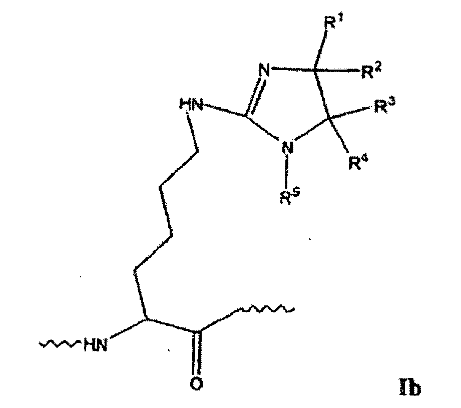 R¹, R², R³ et R⁴ sont chacun indépendamment un membre sélectionné dans le groupe se composant de hydrogène, deutérium, halogène, hydroxyle, alkyle, alkoxy, aryle et d'un marqueur d'affinité, ou, dans une variante, R², R³ et les carbones auxquels ils sont attachés se joignent pour former un noyau carbocyclique, hétérocyclique, aryle ou hétéroaryle à *n* chaînons,
*n* est un entier de 4 à 8,
R⁵ est un membre sélectionné dans le groupe se composant de hydrogène, halogène, hydroxyle, alkyle, alkoxy, et aryle de façon à former un polypeptide ionisé,
X est un hétéroatome sélectionné dans les groupes se composant de O et S,
y est 0, et
b) l'incubation d'un deuxième échantillon dudit polypeptide contenant de la lysine avec une deuxième version isotopique d'un composé de la Formule IIa de façon à former un deuxième polypeptide modifié possédant un résidu lysine de la Formule Ib,
c) la combinaison dudit premier échantillon et dudit deuxième échantillon dudit polypeptide modifié possédant un résidu lysine de la Formule Ib de façon à former un mélange, et
d) l'ionisation dudit mélange de façon à former une série de paires de polypeptides isotopiquement marquées, où les concentrations relatives dudit premier polypeptide modifié possédant un résidu lysine de la Formule Ib et dudit deuxième polypeptide modifié possédant un résidu lysine de la Formule Ib sont directement proportionnelles à leurs intensités de signal relatives, et où la différence de masse entre les deux polypeptides modifiés indique le nombre de lysines qui sont présentes dans ledit polypeptide contenant de la lysine.

14. Le procédé d'exécution d'une quantification différentielle d'un polypeptide contenant de la lysine selon la revendication 13, où ladite série de paires de polypeptides isotopiquement marquées indique qu'au moins deux lysines sont présentes.

15. Le procédé d'exécution d'une quantification différentielle d'un polypeptide contenant de la lysine selon la revendication 13 ou 14, où ledit premier échantillon et ledit deuxième échantillon desdits polypeptides contenant de la lysine sont combinés dans des proportions égales.

16. Un procédé de détermination du nombre de lysines dans un polypeptide contenant de la lysine, ledit procédé comprenant :
a) l'incubation d'un échantillon d'un polypeptide contenant de la lysine avec une première version isotopique d'un composé de la Formule IIa et une deuxième version isotopique d'un composé de la Formule IIa de façon à former un mélange possédant un premier polypeptide modifié possédant un résidu lysine de la Formule Ib et un deuxième polypeptide modifié possédant un résidu lysine de la Formule Ib, où la Formule Ib est la suivante : 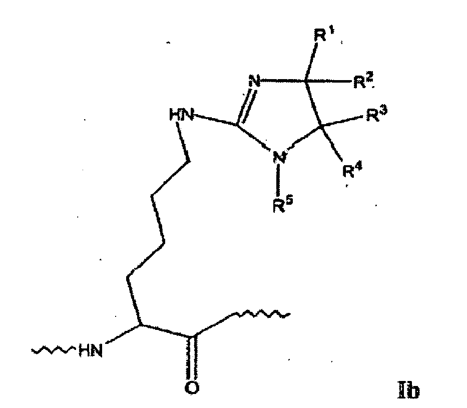 R¹, R², R³ et R⁴ sont chacun indépendamment un membre sélectionné dans le groupe se composant de hydrogène, deutérium, halogène, hydroxyle, alkyle, alkoxy, aryle et d'un marqueur d'affinité, ou, dans une variante, R², R³ et les carbones auxquels ils sont attachés se joignent pour former un noyau carbocyclique, hétérocyclique, aryle ou hétéroaryle à *n* chaînons,
*n* est un entier de 4 à 8,
R⁵ est un membre sélectionné dans le groupe se composant de hydrogène, halogène, hydroxyle, alkyle, alkoxy, et aryle de façon à former un polypeptide ionisé, et
b) l'ionisation dudit mélange de façon à former une série de paires de polypeptides isotopiquement marquées, où la différence de masse entre ledit premier polypeptide modifié possédant un résidu lysine de la Formule Ib et ledit deuxième polypeptide modifié possédant un résidu lysine de la Formule Ib indique le nombre de lysines qui sont présentes dans ledit polypeptide contenant de la lysine.

17. Le procédé de détermination du nombre de lysines dans un polypeptide contenant de la lysine selon la revendication 16, où ladite série de paires de polypeptides isotopiquement marquées indique qu'au moins deux lysines sont présentes.
